(19) Europäisches Patentamt
European Patent Office
Office européen des brevets

(11) EP 1 095 071 B1

(12) **EUROPEAN PATENT SPECIFICATION**

(45) Date of publication and mention of the grant of the patent:
**16.06.2004 Bulletin 2004/25**

(21) Application number: **99930717.6**

(22) Date of filing: **25.06.1999**

(51) Int Cl.[7]: **C08F 12/30**, C07C 317/18, C08G 63/688, C07C 311/24, C07C 309/11, H01M 6/18, H01M 10/40, H01B 1/12

(86) International application number:
**PCT/US1999/014397**

(87) International publication number:
**WO 1999/067304 (29.12.1999 Gazette 1999/52)**

(54) **AROMATIC POLYMERS WITH PENDANT FLUORINATED IONIC GROUPS**
AROMATISCHE POLYMERE MIT FLUORIERTEN IONISCHEN GRUPPEN
POLYMERES AROMATIQUES PORTANT DES GROUPES IONIQUES FLUORES PENDANTS

(84) Designated Contracting States:
**DE FI FR GB NL**

(30) Priority: **25.06.1998 US 90620 P**
**17.07.1998 US 93226 P**

(43) Date of publication of application:
**02.05.2001 Bulletin 2001/18**

(73) Proprietor: **E.I. DUPONT DE NEMOURS AND COMPANY**
**Wilmington, Delaware 19898 (US)**

(72) Inventors:
- **DOYLE, Christopher, Marc**
**Newark, DE 19711 (US)**
- **FIERING, Andrew, Edward**
**Wilmington, DE 19807 (US)**
- **CHOI, Susan, Kuharcik**
**Downingtown, PA 19335 (US)**

(74) Representative: **Matthews, Derek Peter et al**
**Frank B. Dehn & Co.,**
**European Patent Attorneys,**
**179 Queen Victoria Street**
**London EC4V 4EL (GB)**

(56) References cited:

| | |
|---|---|
| **DE-A- 3 828 063** | **DE-A- 19 527 362** |
| **GB-A- 2 051 831** | **US-A- 5 463 005** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 095 071 B1

Printed by Jouve, 75001 PARIS (FR)

## Description

### FIELD OF THE INVENTION

This invention provides a new class of unsaturated compounds including those containing a fluoroether-substituted aromatic ring, polymers, including ionomers, formed therefrom, and processes for forming them. The compositions of the invention are suitable for use in electrochemical applications.

### BACKGROUND OF THE INVENTION

Polymers containing non-aromatic pendant fluoroalkylsulfonic acids and the salts thereof with univalent metals are available commercially as Nafion® Perfluoroionomer available from E. I. du Pont de Nemours and Company, Wilmington DE. Polymers containing non-aromatic pendant fluorosulfonyl imides and fluorosulfonyl methides and the salts thereof with univalent metals are disclosed in DesMarteau (U.S. Patent 5,463,005) wherein they are attached to a perfluorinated backbone.

Narang et al. (U.S. Patent 5,633,098) discloses polysiloxanes and polyacrylates which have fluorinated poly (alkylene oxide) side chains with associated ionic species. In one embodiment, the side chains have the structure $-(CH_2)_{x1}(OCH_2CH_2)_{y1}(OCF_2CF_2)_{z1}SO_2R^3$ wherein $R^3$ is OM, $N(M)SO_2CF_3$ or $C(M)(SO_2CF_3)_2$ and M is an alkali metal.

Armand et al. (U.S. Patent 5,627,292) disclose monomers of the formula $AXFCSO_2Z$ wherein A is $R^3$ or $R^3OCF_2$-, X is F, Cl, H or a perfluoroalkyl group, Z is an ionic group and $R^3$ is a nonperfluorinated polymerizable group. Polymers containing pendant $-CH_2OCF_2CF_2SO_2F$ groups are disclosed by Hamel and Gard, J. Fluorine Chem., volume 68, pages 253-259 (1994). Benrabah et al., J. Power Sources, volume 54, pages 456-460 (1995) disclose ionically conducting polymers prepared from the monomers $R^1R^2NC(O)CF(CF_3)SO_3Li$ wherein $R^1$ and $R^2$ are allyl or $R^1$ is allyl and $R^2$ is methyl.

The above references do not disclose compounds containing a fluorinated ionic group attached directly to an aromatic ring by the thermally and chemically stable ether linkage.

Reactions of phenol salts with $BrCF_2CF_2Br$ to form $ArOCF_2CF_2Br$ are disclosed in Clement et al. (U.S. Patent 5,037,919). Reactions of fluoroalkyl bromides and iodides with sodium dithionite or other sulfinating reagents to form fluoroalkylsulfinates are disclosed in Chemical Abstracts 105:208423j. Conversions of fluoroalkylsulfinate salts to fluoroalkylsulfonyl chlorides and fluorides are disclosed by Hu and DesMarteau, Inorg. Chem. Volume 32, pages 5007 to 5010 (1993). Synthesis of fluoroalkyl sulfonates and sulfonyl imides from the corresponding fluoroalkylsulfonyl halides is also known, as disclosed for example by Waddell et al. (U.S. Patent 5,514,493) and DesMarteau (U.S. Patent 5,463,005).

Reactions of fluorinated vinyl ethers with phenolic compounds in the presence of base are known, as disclosed for example by Fuss and Hintzer, Ger. Offen DE 3 828 063 (1990) and Meazza et al., Eur. Pat. Appl. EP 0 293 943 (1988), and Feiring and Wonchoba (J. Org. Chem. Volume 57, pages 7015-7017). Feiring, U.S. Patent 5,198,570 (1993) discloses the synthesis of aryloxy-fluoroether esters of structure $Ar(OCF_2CFHOR^1CO_2R^2)_p$ wherein Ar is an organic radical containing one or more aromatic rings, each $R^1$ is perfluoroalkyl or ether, thioether, chloro, hydrogen, alkyl or phenyl substituted perfluoroalkyl, and p is 1 to 5 which are prepared by reaction of phenolic compounds with fluorine containing olefins of structure $CF_2{=}CFOR^1CO_2R^2$ in the presence of a base.

Inukai et al. (JP 3-230169) disclose homopolymers and copolymers having monomer units of the formula

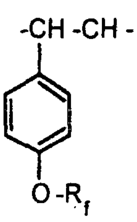

where $R_f$ is perfluoroalkenyl having 3-12 carbons, comonomers including styrene and ethylene. The polymers are said to be formed by subjecting polymers having vinyl phenol units to reaction with a perfluoroolefin oligomer in the presence of a basic catalyst in a nonaqueous solvent.

SUMMARY OF THE INVENTION

The present invention provides for a polymer comprising pendant groups comprising the radical described by the formulas Ia or Ib:

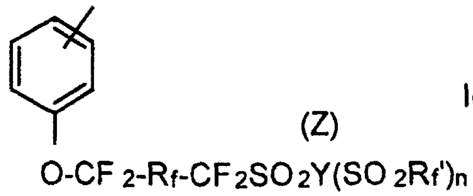

I(a))

or

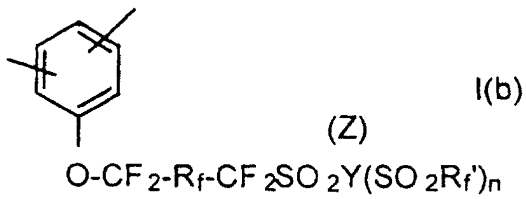

I(b)

wherein $R_f$ is a bond or is a fluoroalkylene group of from 1 to about 10 carbon atoms, optionally substituted by one or more ether oxygens and one or more hydrogen atoms. Y is N, O, C, $R_f'$ is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

Further provided are compounds described by the formula (II)

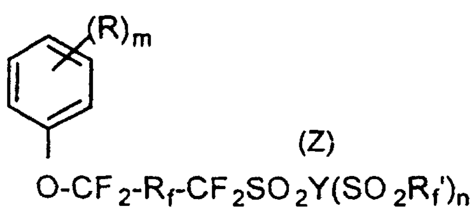

(II)

wherein m = 0, 1 or 2 and when m = 1 R is a polymerizable group or bromo or iodo, and when m = 2, R represents polymerizable groups or bromo or iodo groups, which are optionally the same, $R_f$ is a bond or is a fluoroalkylene group of from 1 to about 10 carbon atoms, optionally substituted by one or more ether oxygens and one or more hydrogen atoms, Y is N, O, C, $R_f'$ is a fluoroalkyl group of from 1 to about 10 carbon atoms, optionally substituted by one or more ether oxygens and one or more hydrogen atoms, n = 0, 1 or 2 n = 0, 1 or 2, with the proviso that n = 0 when Y = O, n = 1 when Y = N and n = 2 when Y = C, and Z is hydrogen or a univalent metal. R is preferably a para positioned polymerizable group, a para bromo group or a para iodo group.

Further provided is a process comprising:

reacting alkali metal salts of substituted phenols described by the formula (III) with 1,2-dibromotetrafluoroethylene to make compounds described by the formula (IV)

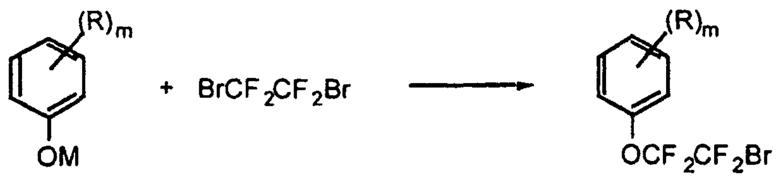

(III) (IV)

wherein m is 0, 1 or 2 and R is bromo, iodo. $CO_2R'$ or $NO_2$, R' is an alkyl group of 1 to 10 carbon atoms and M is an alkali metal.

reacting the compound described by formula (IV) with a sulfinating reagent to form an alkali metal sulfinate described by structure (V)

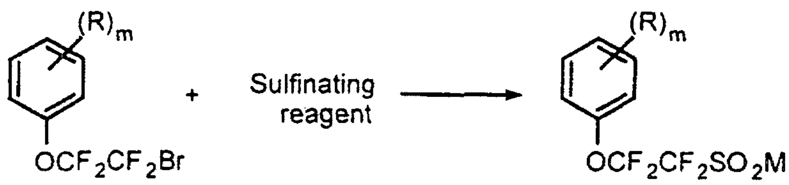


(IV) (V)

Reacting the alkali metal sulfinate of structure (V) with elemental chlorine or bromine to give the corresponding sulfonyl chloride or bromide described by structure (VI) wherein X = Cl or Br;

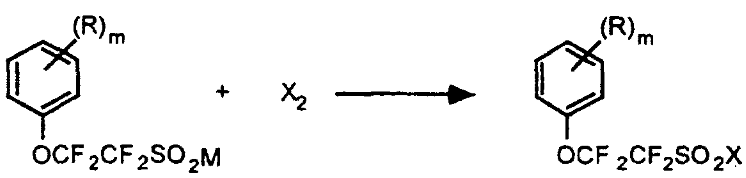

(V) (VI)

Further provided is an ionically conductive composition comprising the ionomer of the invention and a liquid imbibed therewithin.

Further provided is an ionically conductive composition comprising the compound described by the formula (II) and a liquid.

Further provided is an ion exchange membrane comprising an ionomer comprising pendant groups comprising the radical described by the formulas I(a) and I(b):

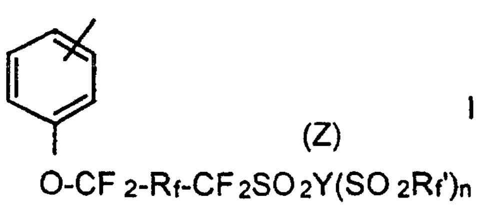

(Z) I(a))

or

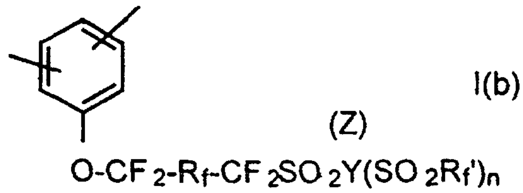

I(b)

wherein $R_f$ is a bond or is a fluoroalkylene group of from 1 to about 10 carbon atoms, optionally substituted by one or more ether oxygens and one or more hydrogen atoms, Y is N, O, or C, $R_f'$ is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

Further provided are electrochemical cells comprising a cathode, an anode and a separator, at least one of which comprises an ionomer comprising pendant groups comprising the radical described by the formulas I(a) and/or I(b)

Further provided is an electrochemical cell comprising an anode, a cathode, a separator, and a conductive composition comprising the compound described by the formula (II) and a liquid.

Further provided is an electrode comprising an electroactive material and an ionomer comprising pendant groups comprising the radical described by formula I(a) and/or I(b).

DETAILED DESCRIPTION OF THE INVENTION

The ionomers of the present invention exhibit high solubility in numerous organic solvents which facilitates the fabrication of thin films and other shaped articles. Relatively low fluorine content permits the use of low cost starting materials, and improves stability in highly reducing environments such as are found in high voltage batteries such as lithium batteries. In contrast with the partially fluorinated ionomers taught in the art, the ionomeric group in the ionomers of the present invention is attached to the polymer backbone by aryl fluoroalkylether linkages which have high stability to strongly acidic or alkaline conditions.

The preferred styrene-based monomers of the invention are especially versatile materials for the synthesis of ionomers with a wide variety of properties because styrenic monomers can be homopolymerized and copolymerized with a wide variety of comonomers and are known to be polymerizabile by free radical, cationic, anionic and coordination polymerization methods. Thus, one skilled in the art may select from many comonomers and polymer structures, including block and graft copolymers, to obtain the desired combination of polymer properties.

The present invention provides for a polymer comprising pendant groups comprising the radical described by the formulas I(a) and I(b):

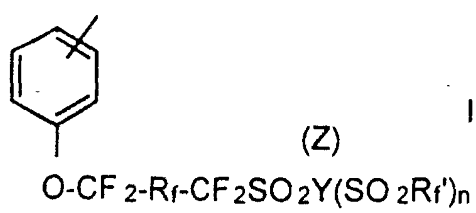

I(a))

or

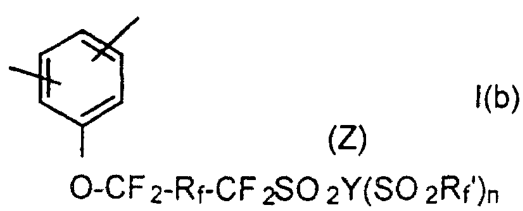

I(b)

wherein $R_f$ is a bond or is a fluoroalkylene group of from 1 to about 10 carbon atoms, optionally substituted by one or more ether oxygens and one or more hydrogen atoms, Y is N, O, C, $R_f$' is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

$R_f$ is preferably a bond, $CFHOCF_2CF(CF_3)OCF_2$ or $CFHOCF_2$; most preferably $R_f$ is a bond. Y is preferably N or O, that is, n = 0 or 1, and Z preferably is a lithium cation. When Y is N or C, $R_f$' is preferably $CF_3$ or $C_2F_5$, most preferably $CF_3$. Preferably the polymer is a polyester or polyolefin, most preferably a polyethylene, having pendant groups comprising the radical described by the formula (I).

The polymer of the invention is preferably formed by polymerization of a monomer described by the formula

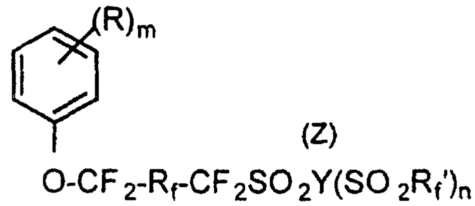

(II)

wherein m = 0, 1 or 2 and when m = 1 R is a polymerizable group or bromo or iodo, and when m = 2, R represents polymerizable groups which are optionally the same, $R_f$ is a bond or is a fluoroalkylene group of from 1 to about 10 carbon atoms, optionally substituted by one or more ether oxygens and one or more hydrogen atoms, Y is N, O, C, $R_f$' is a fluoroalkyl group of from 1 to about 10 carbon atoms, optionally substituted by one or more ether oxygens and one or more hydrogen atoms, n = 0, 1 or 2 n = 0, 1 or 2, with the proviso that n = 0 when Y = O, n = 1 when Y = N and n = 2 when Y = C, and Z is hydrogen or a univalent metal. R is preferably a para positioned polymerizable group, a para bromo group or a para iodo group. $R_f$ is preferably a bond, $CFHOCF_2CF(CF_3)OCF_2$ or $CFHOCF_2$; most preferably $R_f$ is a bond. Y is preferably N or O, that is, n = 0 or 1, and Z is preferably a lithium cation. When Y is N, $R_f$' is preferably $CF_3$ or $C_2F_5$, most preferably $CF_3$. $(R)_m$ is preferably 4-$CH=CH_2$ when m = 1 or 3,5-di-$CO_2CH_3$ when m = 2. Most preferably, R is 4-$CH=CH_2$ and m = 1.

The preferred embodiment of the monomer of the invention, wherein $R_f$ is a bond, is preferably formed by the process of the invention, the process comprising

reacting alkali metal salts of substituted phenols described by the formula (III) with 1,2-dibromotetrafluoroethylene to make compounds described by the formula (IV)

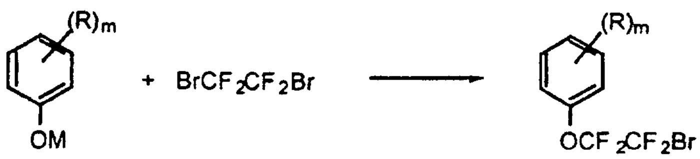

(III) (IV)

wherein in (III), m is 0, 1 or 2 and (R) is a polymerizable group or a precursor thereof and is preferably a bromo, iodo, diester or dinitro compound, and wherein when m = 2, R groups are optionally the same, and

reacting the compound described by the formula (IV) with a sulfinating reagent such as sodium dithionite to form an alkali metal sulfinate described by the formula (V)

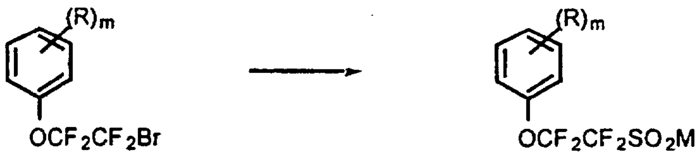

(IV) (V)

In one embodiment of the invention, the compound described by the formula (V) is converted to the corresponding sulfonyl chloride or bromide by reaction with elemental chlorine or bromine, represented by the formula

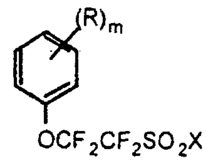

(VI)

wherein X is bromine or chlorine, preferably chlorine. The compound described by the formula (VI), in turn may be subject to hydrolysis under basic conditions to form alkali metal, preferably lithium, sulfonates described by the formula (VII)

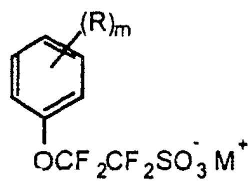

(VII)

where $M^+$ is an alkali metal. The composition described by the formula (VII) when m = 2 has ester groups, preferably $-COOCH_3$, at the 3,5 positions, and may be polymerized or copolymerized by condensation polymerization reactions as are known in the art to form a polyester. The bromo and iodo substituents are preferably in a para position. When m = 1, the bromo or iodo substituent may be converted to a polymerizable alkenyl group, preferably vinyl, according to the methods taught in R. F. Heck, Acc. Chem. Res., volume 12, pages 146-51 (1979), the resulting styrenic monomer is then homo or copolymerized by means known in the art to form an ionomer of the invention.

It is possible to fluorinate the brominated or iodated monomer to form the corresponding sulfonyl fluoride prior to hydrolysis, but this additional step is not necessary.

In a preferred embodiment of the process of the invention, the sulfonyl chloride or bromide described by the formula (VI) is exposed to an ionic fluoride, preferably an alkali metal fluoride, to form the compound described by the formula (VIII). The compound described by the formula (VIII) is then reacted with perfluoroalkylsulfonamides under basic conditions, preferably alkali metal containing base, to form sulfonylimide compounds described by the formula (IX), as shown:

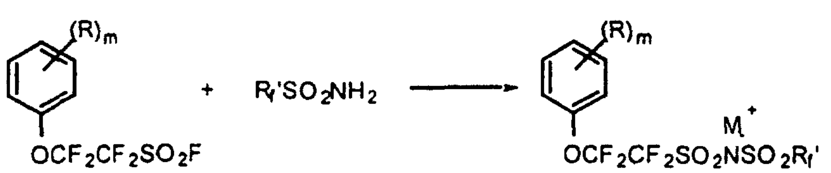

(VIII) (IX)

In direct analogy to the case of the compound described by the formula (VII), in the composition described by the formula (IX) when m = 2, ester groups, preferably $-COOCH_3$, are disposed at the 3,5 positions, and may be polymerized or copolymerized by condensation polymerization reactions as are known in the art to form a polyester. When m = 1, the bromo or iodo substituents, preferably in the para form, may be converted to a polymerizable alkenyl group, preferably vinyl, according to the methods taught in R. F. Heck, Acc. Chem. Res., volume 12, pages 146-51 (1979), the resulting styrenic monomer is then homo or copolymerized to form an ionomer of the invention.

The foregoing processes are directed at forming the monomers and related polymers, and ultimately the ionomers which are preferred in the practice of the present invention, namely those wherein $R_f$ is a bond.

Other monomers and polymers of the invention, wherein $R_f$ is not a bond, may be formed by other processes.

For example, phenols may be reacted with fluorinated olefins described by the formula (X) in the presence of a catalytic amount of base according to the following equation:

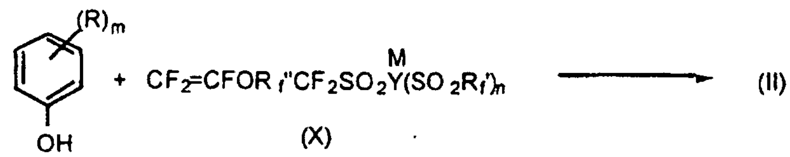


(X)

In the compound described by the formula (X) $R_f$" is a fluoroalkylene group of from one to ten carbon atoms optionally substituted by one or more ether oxygens, M is an alkali metal and $(R)_m$, Y, $R_f$' and n are as described above. This process provides compounds of structure (II) in which $R_f$ is CHFOR$_f$", that is $R_f$ contains at least one hydrogen.

In another process, a phenolic compound is reacted with an iodo-substituted fluoroalkylacyl chloride to form an ester described by the formula (XI). The resulting ester is reacted with sulfur tetrafluoride to form an ether described by the formula (XII). Reaction of the fluoroalkyl iodide with a sulfinating reagent such as sodium dithionite provides a sulfinate salt described by the formula (XIII) which could be converted to compounds described by the formula (II) by processes disclosed herein above.

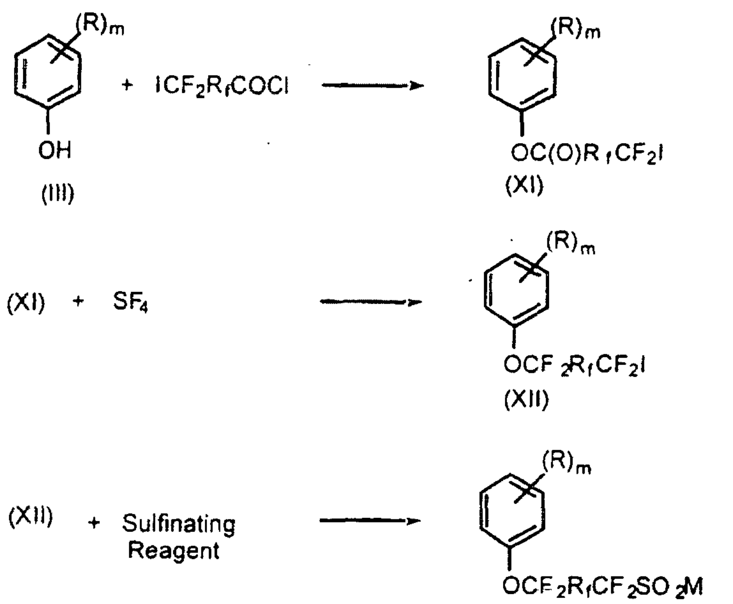


(XII)

In the formulas (XII) $R_f$ is a perfluorofluoroalkylene group having from 1 to about 10 carbon atoms, optionally substituted by one or more ether oxygens.

The R group(s) in the compound described by the formula (II) of the present invention impart polymerizability to the monomers of the invention, or are precursors of groups which impart polymerizability. The goal is to provide the ionomer described by the formula (I) by homo- or copolymerization of one or another of the monomers of the invention preceeded by or followed by all the necessary reactions hereinabove described in order to achieve one or another of the ionomers of the invention.

The choice of R to start with is related not only to the desired final product, but also by the stability of that choice to the intervening reactions between starting material and final ionomer. Ester groups, for example, are stable to the reaction of the substituted phenol (III) with dibromotetrafluoroethylene and the subsequent sulfination of the product thereof (IV) to the metal sulfinate (V). However, vinyl groups are not stable in those reactions. Thus, if a poly vinyl ionomer is the desired final product, it is desirable to start with structure (III) wherein R is a convenient precursor for the vinyl group such as bromine or iodine which can be converted, after the formation of structure (VII or IX), to the vinyl group by the Heck reaction, op. cit. In another example, structure (II) with R = 3,5-dinitro may be reduced to R =

3,5-diamino for the synthesis of polyamides and with R = 4-cyano may be converted to R = 4-(2-oxazoline) for the synthesis of polyoxazolines.

Compounds of structure (II) with R = 3- or 4-ethenyl, that is, substituted styrene monomers, are especially preferred. 3- or 4-Hydroxystyrenes are known compounds and can be reacted with compound (X) to form the desired derivatives. However, 3- and 4-hydroxystyrenes are expensive and not readily available and the olefinic group may interfere with steps in the process using intermediates IV, V, VI and VIII. Thus, it may be preferable to conduct the above reactions on the more readily available 3 or 4-bromo or iodophenols and subsequently convert the bromo or iodo groups to $CH=CH_2$ by reaction with ethylene and a palladium catalyst.

Polymers containing the radical described by the formula (I) are prepared from monomers (II) by a variety of well-known techniques which depend on the exact structure of the monomeric compound and the properties desired for the final product. Thus, monomers (II) in which R is $-CH=CH_2$ are especially preferred because such styrenic compounds are readily homopolymerizable by free radical, anionic, cationic or coordination polymerization techiques. Such styrenic derivatives may also be copolymerized with other monomers to make copolymers in which units of structure (I) comprise from about 1% to about 99% of the repeat units in such polymers. Examples of other monomers include styrene, substituted styrenes, acrylonitrile, alkyl and aryl methacrylates, alkyl and aryl acrylates, acrylamides, isoprene, chloroprene, butadiene, vinyl acetate, N-vinylpyrrolidinone and mixtures of such comonomers. Both random and block co (ter)polymers may be produced by application of well known polymerization techniques. The ionomers of the present invention may optionally be crosslinked by techniques known to those skilled in the art.

Crosslinking may be advantageous by allowing the resulting shaped polymer article, such as a film, to swell but not dissolve in various solvents. To prepare crosslinked materials, monomers (II) in which R is $-CH=CH_2$, for example, may be mixed with a di- or trifunctional monomer, such as poly(ethylene glycol) diacrylate or trimethylolpropane triacrylate, an initiator and, optionally, other comonomers. A solvent, such as DMF, may be added to form a homogeneous mixture which can be cast and heated to cause polymerization to a crosslinked polymer film. Crosslinked films may also be obtained by mixing monomers (II) in which R is $-CH=CH_2$ with a polymer containing unsaturated groups and a radical initiator. The ingredients may be blended in the melt or in a solvent such as DMF, formed into a film or other shaped article and heated so as to effect crosslinking. A representative polymer with unsaturated groups is Hydrin T, a terpolymer of ethylene oxide, epichlorohydrin and allyl glycidyl ether, available from Zeon Chemicals Incorporated, Hattiesburg, MS.

Monomers (II) in which the R groups are two methyl ester units will undergo condensation polymerizations with diols to form polyesters containing units of structure (I). Suitable diols include ethylene glycol, 1,3-propanediol, 1,4-butanediol, polyethylene glycols, poly(tetramethylene ether)glycols. hydroquinone and substituted hydroquinones. Mixtures of diols may be employed. In addition, other diester monomers, such as dimethyl terephthalate, may be used with the diester monomer (II) to afford copolymers. It is apparent that a wide variety of condensation polymers, including polyesters, polyamides and polycarbonates may be prepared which contain units of structure (I) by appropriate choice of monomers.

While there is no limit to the shape or proportions of an article formed from the ionomers of the invention, thin films or membranes are of particular utility as separators in electrochemical cells, preferably in lithium ion batteries. In some cases it may be possible to extrude films using a screw extruder and a flat die. Alternatively, films can be melt pressed. In an additional embodiment, films may be cast from solutions or dispersions of the polymers by casting onto a substrate and coagulating. No particular method is preferred over another, and the specific method will be chosen according to the needs of the particular practitioner.

Additives may be included to improve the property of the separator materials and separators may also be formed by addition of the ionomers herein to porous substrates.

The ionomers of the present invention exhibit room temperature ionic conductivity of ca. $10^{-7}$-$10^{-6}$ S/cm when dry. However, it is found in the practice of the invention that numerous liquids when imbibed into the ionomer of the invention enhance the conductivity by orders of magnitude. Thus it has been found desirable in order to achieve the most useful embodiments of the present invention to form conductive compositions wherein liquids are imbibed into an ionomer of the invention.

The liquid employed will be dictated by the application. In general terms, it has been found in the practice of the invention that conductivity of the liquid-containing ionomer increases with increasing % weight uptake, increasing dielectric constant, and increasing Lewis basicity of the liquid, while conductivity has been observed to decrease with increasing viscosity and increasing molecular size of the liquid employed. Thus, a highly basic solvent of low viscosity and small molecular size but low dielectric constant may provide superior conductivity in a given membrane than a larger, more viscous, less basic solvent of very high dielectric constant. Of course, other considerations come into play as well. For example, excessive solubility of the ionomer in the liquid may be undesirable, or, the liquid may be electrochemically unstable in the intended use.

One particularly preferred embodiment comprises the lithium ionomer combined with aprotic solvents, preferably organic carbonates or diesters, including mixtures thereof, which are useful in lithium batteries. Most preferably,

the liquid is a mixture of ethylene carbonate and dimethyl succinate.

Among the uses for the present invention are as fuel cells, sensors, electrochemical capacitors, primary and rechargeable batteries and other electrochemical device applications.

It is further found in the practice of the invention that useful conductive compositions are formed by forming solutions of the salts described by the formula (II). $R_f$ is preferably a bond, $CFHOCF_2CF(CF_3)OCF_2$ or $CFHOCF_2$; most preferably $R_f$ is a bond. Y is preferably N or O, that is, n = 0 or 1, and Z is preferably a lithium cation. When Y is N, $R_f'$ is preferably $CF_3$ or $C_2F_5$, most preferably $CF_3$.

Solvents suitable for use in forming the conductive solutions herein include water, alcohols, and aprotic organic liquids. Preferably, the solvents are organic carbonates, with mixtures of ethylene carbonate and dimethyl carbonate EC/DMC most preferred.

The preferred electrode of the invention comprises a mixture of one or more electrode active materials in particulate form, an ionomer of the invention, at least one electron conductive additive, and at least one organic carbonate. Examples of useful anode active materials include, but are not limited to, carbon (graphitic, coke-type, mesocarbons, polyacenes, and the like) and lithium-intercalated carbon, lithium metal nitrides such as $Li_{2.6}Co_{0.4}N$, tin oxide-based glasses, lithium metal, and lithium alloys, such as alloys of lithium with aluminum, tin, magnesium, silicon, tin, manganese, iron, and zinc. Lithium intercalation anodes employing carbon are preferred. Useful cathode active materials include, but are not limited to, transition metal oxides and sulfides, lithiated transition metal oxides and sulfides, and organosulfur compounds. Examples of such are cobalt oxides, manganese oxides, molybdenum oxides, vanadium oxides, sulfides of titanium, molybdenum and niobium, lithiated oxides such as spinel lithium manganese oxides $Li_{1+x}Mn_{2-x}O_4$, chromium-doped spinel lithium manganese oxides $Li_xCr_yMn_zO_4$, $LiCoO_2$, $LiNiO_2$, $LiNi_xCo_{1-x}O_2$ where x is $0 < x < 1$, with a preferred range of $0.5 < x < 0.95$, $LiCoVO_4$, and mixtures thereof. $LiNi_xCo_{1-x}O_2$ is preferred. A highly preferred electron conductive aid is carbon black, preferably Super P carbon black, available from the MMM S.A. Carbon, Brussels, Belgium, in the concentration range of 1-10%. Preferably, the volume fraction of the lithium ionomer in the finished electrode is between 4 and 40%.

The preferred electrode of the invention may conveniently be made by dissolution of all polymeric components into a common solvent and mixing together with the carbon black particles and electrode active particles. For cathodes the preferred electrode active material is $LiNi_xCo_{1-x}O_2$ wherein $0 < x < 1$, while for anodes the preferred electrode active material is graphitized mesocarbon microbeads. For example. a preferred lithium battery electrode of the invention can be fabricated by dissolving ionomer of the invention in admixture of acetone and dimethylformamide, followed by addition of particles of electrode active material and carbon black, followed by deposition of a film on a substrate and drying. The resultant preferred electrode will comprise electrode active material, conductive carbon black, and ionomer of the invention, where, preferably, the weight ratio of ionomer to electrode active material is between 0705 and 0.8 and the weight ratio of carbon black to electrode active material is between 0.01 and 0.2. Most preferably the weight ratio of ionomer to electrode active material is between 0.1 and 0.25 and the weight ratio of carbon black to electrode active material is between 0.02 and 0.1. This electrode can then be cast from solution onto a suitable support such as a glass plate or current collector metal foil, and formed into a film using techniques well-known in the art. The electrode film thus produced can then be incorporated into a multi-layer electrochemical cell structure by lamination, as hereinbelow described. Other embodiments may be made by methods known to those skilled in the art. See, for example, descriptions of lithium-ion cell fabrication procedures in U.S. Patent No. 5,658,683(Sony Corp. Aug. 19, 1997) and U.S. Patent No. 4,668,595 (Asahi May 26, 1987).

It may be desirable to incorporate into the electrode composition of the invention such adjuvants as may be useful for such purposes as improving the binding of the components thereof, or providing improved structural integrity of an article fabricated therefrom. This improvement may be accomplished by incorporating 2-5% by weight of a polymeric binder. One particularly preferred additional material is polyvinylidene fluoride which may be incorporated simply by dispersing the particles thereof into the same solution from which the electrode is being formed, as hereinabove described.

In an alternative process, the dispersion of electrode-active material and optional carbon black and other adjuvants can first be cast onto a surface followed by addition of the ionomer of the invention in organic carbonate solution.

In a preferred embodiment of the battery of the present invention, a battery is formed from one or more electrochemical cells formed by laminating together in film form the anode, cathode, and separator compositions of the present invention, all of which have been rigorously dried prior to addition of a liquid selected from the group of organic carbonates and mixtures thereof, a mixture of ethylene carbonate and dimethyl carbonate being most preferred.

The relatively high solubility of the ionomers of the present invention provides a benefit in ease of processing during fabrication of the components of a battery but may be problematical during final assembly of the desired battery product. Organic carbonates will not only swell the ionomeric polymer, but may also dissolve the polymer depending on the composition thereof, the primary determining factor being the degree of crystallinity, which in turn is related to the concentration of ionic comonomer in the polymer. The challenge is to swell the ionomer with solvent while minimizing

dissolution of the polymer.

It may thus be desirable to enhance the physical properties of the solvent-swollen membrane. Means available for improving the mechanical properties include: 1) Incorporation into the polymer by means known in the art a non-ionic comonomer that is less solvent sensitive; 2) formation by known means of a polymer blend with a non-ionic polymer that is less solvent sensitive; 3) blending by known means of the ionomer of the invention with an inert filler; 4) blending different compositions of ionic copolymers; and 5) cross-linking.

A preferred method is to blend the ionomer with an inert filler prior to formation of the separator membrane or film. Suitable inert fillers include $SiO_2$, $Al_2O_3$, $TiO_2$, or $CaF_2$, with $SiO_2$ preferred. Small and high surface area particles less than 1.0 micron in diameter are desired, such as are available for the preferred grade of $SiO_2$ under the trade name Cab-o-sil® TS-530 silica. Loadings of up to 50 weight % filler are preferred.

Another approach comprises dissolution of the ionomer into the preferred organic carbonate solvents, followed by introduction of the resulting solution or gel into the pores of an inert porous polymer support such as Celgard® porous polypropylene, available from Hoechst-Celanese, or Gore-Tex microporous PTFE, available from W.L. Gore Associates, Newark, DE.

In alternative embodiments of the invention, a non-polymeric salt described by the formula (II) may be employed in addition to or in place of the ionomer of the invention as it is employed the embodiments hereinabove described.

The invention is further described in the following specific embodiments.

EXAMPLES

For the purposes of this invention, the term "conductivity" used herein refers specifically to ionic conductivity as determined using the so-called four-point probe technique described in an article entitled " Proton Conductivity of Nafion® 117 As Measured by a Four-Electrode AC Impendance Method" by Y. Sone et al., J. Electrochem. Soc., 143, 1254 (1996). The method as described applies to aqueous electrolyte membranes. The method was modified for purposes of obtaining the measurements reported herein for non-aqueous solvents by placing the apparatus described in a sealed glove box purged with dry nitrogen in order to minimize any exposure to water. The method was also modified by substituting parallel linear probes traversing the full width of the test specimen for the point probes employed in the published method.

A 1.0 cm by 1.5 cm film was blotted dry and positioned into the conductivity cell. Cell impedance was determined over the range of 10 Hz to 100,000 Hz, and the value with zero phase angle in the higher frequency range (usually 500-5000 Hz) was ascribed to the bulk sample resistance in Ohms. The raw resistance value was then converted to conductivity, in S/cm, using the cell constant and film thickness.

Solvent uptake was determined from the equation

$$\% \text{ uptake} = (W_w - W_d)/W_d$$

where $W_d$ was the weight of the membrane prior to solvent contact and $W_w$ was the weight of the membrane after solvent contact determined after first removing membrane from solvent and then blotting it dry using a paper towel to remove excess surface solvent.

All chemicals were used as received unless stated otherwise.

$^{19}$F NMR spectra were recorded using a Bruker AVANCE DRX 400 spectrometer. $^{1}$H NMR spectra were recorded using a Bruker AVANCE DRX 500 spectrometer.

EXAMPLE 1

Synthesis of 2-(4-Bromophenoxy)tetrafluoroethanesulfonyl Chloride

4-Bromophenol (Aldrich Chemicals, 348.4 g, 2.01 mol) was dissolved in 1.95 L of 1.033 N potassium hydroxide in methanol. This solution was evaporated to dryness on a rotary evaporator and the resulting solid was dried at 140°C and 0.1 mm. The solid was mixed with 600 mL of DMSO under nitrogen. 1,2-Dibromotetrafluoroethane (571.6 g, 2.2 mol) was added dropwise at 30-40°C. The resulting mixture was heated to 60°C for 6 hr. It was cooled to room temperature and diluted to 3-L with ice and water. The organic layer was separated and the aqueous solution was extracted with 2 X 75 mL of methylene chloride. The methylene chloride extracts were concentrated on a rotary evaporator and the residue combined with the original organic layer. This material was washed with 2 X 400 mL of water, dried over anhydrous magnesium sulfate and filtered. The filtrate was distilled giving 641.2 g (92%) of 1-bromo-2-(4-bromophenoxy)tetrafluoroethane, bp 57°C at 0.6 mm. This product from a similar preparation showed $^{1}$H NMR ($\delta$, $CDCl_3$) 7.1

(d, 2H), 7.5 (d, 2H); $^{19}F$ NMR (δ, $CDCl_3$) -68.6 (2F), -86.6 (2F). Anal. Calcd. for $C_8H_4F_4OBr_2$: C, 27.30; H, 1.15; Br, 45.41; F, 21.59. Found: C, 27.27; H, 1.16; Br. 44.85; F, 20.87.

A 366.5 (1.04 mol) portion of the above product was added under nitrogen to a stirred mixture of 1.026 L of distilled and deoxygenated water, 186.5 g of sodium bicarbonate, 500 mL of dimethylformamide and 357.6 g of sodium dithionite. This mixture was heated to 65°C resulting in a rapid gas evolution. Gas evolution ceased after about 1 hr and the mixture was heated to 70-75°C for 3 hr. It was cooled to about 10°C in an ice water bath and 1-L of ethyl acetate was added. The mixture was filtered and the solid was washed with ethyl acetate. The combined filtrates were separated into aqueous and organic layers and the organic layer was washed with 4 X 50 mL of saturated aqueous sodium chloride solution. The organic layer was concentrated on a rotary evaporator to about 1/4 its initial volume and filtered. The solid was washed with ethyl acetate. The combined organic solutions were concentrated to dryness on a rotary evaporator giving 362.6 g (97%) of white solid sodium 2-(4-bromophenoxy)tetrafluoroethanesulfinate. $^1H$ NMR (δ, $CD_3OD$) 7.2 (d, 2H), 7.6 (d, 2H); $^{19}F$ NMR (δ, $CD_3OD$) -132.8 (2F), -81.9 (2F).

The above product was dissolved in a mixture of 600 mL of deoxygenated water and 300 mL of 1,1,2-trichlorotrifluoroethane in a round bottom flask equipped with a dry ice condenser and cooled to 5-15°C. Chlorine gas (134 g) was bubbled into this mixture over about 1 hr. The resulting yellow mixture was stirred 1 hr without external cooling. It was warmed to 20°C and an additional 200 mL of 1,1,2-trichlorotrifluoro-ethane was added. The organic layer was separated and the aqueous solution was extracted with 100 mL of 1,1,2-trichlorotrifluoroethane. The combined organic solutions were dried over anhydrous magnesium sulfate and concentrated on a rotary evaporator. The residue was distilled through a short Vigreux column giving 361.1 g (97%) of 2-(4-bromophenoxy)tetrafluoroethanesulfonyl chloride, bp 71°C at 0.3 mm. $^1H$ NMR (δ, $CDCl_3$) 7.1 (d, 2H), 7.6 (d, 2H); $^{19}F$ NMR (δ, $CDCl_3$) -79.0 (2F). -107.9 (2F). A sample from a similar preparation was submitted for elemental analysis. Anal. Calcd. for $C_8H_4F_4BrClSO_3$: C, 25.86; H, 1.08; F, 20.46; S, 8.63. Found: C, 26.07; H, 1.17; F, 18.74; S, 8.59.

EXAMPLE 2

Synthesis of Lithium 2-(4-bromophenoxy)tetrafluoroethanesulfonate

Lithium hydroxide monohydrate (57.3 g, 1.365 mol) was dissolved in 600 mL deoxygenated distilled water. THF (150 mL) was added and this solution was heated to 35°C. The heat source was removed and 237 g (0.64 mol) of 2-(4-bromophenoxy)-tetrafluoroethanesulfonyl chloride was added dropwise over 45 min at a rate so that the exotherm maintained the solution at about 55°C. After the addition was complete, the solution was held at 55°C for an additional 1.5 hr. The solution was cooled to room temperature. Its pH was adjusted to 7 by addition of about 3 mL of concentrated hydrochloric acid and the aqueous solution was evaporated to dryness on the rotary evaporator. The solid was slurried with ether and filtered. The ether solution was treated with three volumes of hexane resulting in deposition of a white solid. The solid was filtered off and washed with hexane. The filtrate was evaporated and the residue was again precipitated from ether solution by addition of hexane. The combined precipitates were recrystallized from acetonitrile with cooling in the refrigerator with the filtrate concentrated several times to collect additonal fractions. The combined recrystallize product was dissolved in ether, filter and concentrated on a rotary evaporator. The product was dried at 100°C and 0.1 mm giving 173.9 g (76%) of the title compound as a white solid. $^1H$ NMR (δ, $CD_3OD$) 7.2 (d, 2H), 7.6 (d, 2H); $^{19}F$ NMR (δ, $CD_3OD$) -116.9 (2F), -81.6 (2F). A sample from a similar preparation was submitted for elemental analysis. Anal. Calcd. for $C_8H_4F_4BrLiSO_3$: C, 26.76; H, 1.12; F, 17.83; S, 8.93. Found: C, 26.57; H, 1.26; F, 18.94; S, 8.77.

EXAMPLE 3

Synthesis of Lithium 2-(4-ethenylphenoxy)tetrafluoroethanesulfonate

A 1 L autoclave was charged with 69 g (0.19 mol) of lithium 2-(4-bromophenoxy) tetrafluoroethanesulfonate, 200 mL of acetonitrile, 0.88 g $Pd(OAc)_2$, 2.48 g of tri-o-tolylphosphine and 200 ml of triethylamine. The autoclave was closed, cooled, evacuated and charged with ethylene to 110 psi. The mixture was heated with stirring to 85°C for 24 hr, holding the gas pressure at 120-125 psi by venting or adding ethylene as needed. The mixture was cooled to room temperature and vented to atmospheric pressure. The autoclave contents were recovered using a mixture of acetonitrile and ether to rinse. The mixture was treated with 8.9 g of lithium hydroxide monohydrate in 150 mL of water with vigorous stirring and filtered through celite. The celite was washed with acetonitrile and ether. The combined filtrates were evaporated to dryness at 75-80°C and 5 mm. The residue was extracted with 0.5 L of ether and filtered. The filtrate was diluted with 0.5 L of hexane and the resulting precipitate was collected, precipitated a second time from a mixture of ether and hexane and dried at 65°C and 0.05 mm giving 20.6 g of product. An additional 12.8 g of product was obtained by concentrating the above ether and hexane filtrates and reprecipitating the residue for a total yield of 33.4

g (58%) of the title product. $^1H$ NMR (δ, $CD_3CN$) 5.27 (d, 1H), 5.80 (d, 1H), 6.78 (dd, 1H), 7.27 (d, 2H), 7.51 (d, 2H); $^{19}F$ NMR (δ, $CD_3CN$) -116.6 (2F), -80.8 (2F). Anal. Calcd for $C_{10}H_7F_4LiO_4S$: C, 39.23; H, 2.30; F, 24.82; Li. 2.27; S, 10.47. Found: C, 38.18; H, 2.78; F, 22.23; L1, 2.10; S, 9.55.

EXAMPLE 4

Synthesis of 2-(4-Bromophenyoxy)tetrafluoroethanesulfonyl Fluoride

2-(4-Bromophenyoxy)tetrafluoroethanesulfonyl chloride (130 g, 0.35 mol) was added dropwise to a stirred mixture of 105 g (1.8 mol) oven dried potassium fluoride and 500 mL of acetonitrile under nitrogen at room temperature. After 24 hr at room temperature, a fluorine NMR spectrum showed about an 80% conversion of the sulfonyl chloride to fluoride. The mixture was warmed to 30-35°C and then allowed to stir for 3 days at room temperature. It was filtered and the solid rinsed with acetonitrile. The combined filtrates were concentrated on a rotary evaporator at 40°C and 150 mm and the residue was distilled on a Kugelrohr at 80-85°C and 5 mm into a dry ice cooled receiver. The liquid distillate was distilled through a 12" Vigreux column giving 111.5 g (90%) of the title product as a colorless liquid bp 81-82°C at 4.5 mm. $^1H$ NMR (δ, $CDCl_3$) 7.1 (d, 2H), 7.5 (d, 2H); $^{19}F$ NMR (δ, $CDCl_3$) -81.7(2F), -111.5 (2F). Anal. Calcd. for $C_8H_4F_5BrSO_3$: C, 27.06; H, 1.14; F, 26.75; S, 9.03; Br, 22.5. Found: C, 27.13; H, 1.05; F, 26.88; S, 8.94; Br, 22.35.

EXAMPLE 5

Synthesis of Lithium N-(Trifluoromethanesulfonyl)-2-(4-Bromophenyoxy)tetrafluoroethanesulfonamide

Freshly sublimed trifluoromethanesulfonamide (15.51 g, 0.104 mol) was added to 240 mL of triethylamine which was freshly distilled from lithium aluminum hydride. The mixture was warmed to 40°C to dissolve the solid, then cooled to room temperature. 2-(4-Bromophenyoxy)tetrafluoroethanesulfonyl fluoride (35.7 g, 0.101 mol) was added and solution was heated at 70-75°C for 18 hr. An F NMR spectrum of the solution showed a trace of sulfonyl fluoride remained so the mixture was treated with an additional 1 g of trifluoromethanesulfonamide and heated for 16 hr at 70-75°C. The resulting red mixture was concentrated on the rotary evaporator. The residue was dissolved in methylene chloride, washed three times with water, dried over anhydrous magnesium sulfate and concentrated on the rotary evaporator to 46.24 g of red oil which was the triethylammonium salt of the title product. $^1H$ NMR (δ, $CDCl_3$) 1.32 (t, 9H), 3.20 (q, 6H), 7.13 (d, 2H), 7.5 (m, 3H (aromatic + NH)); $^{19}F$ NMR (δ, $CDCl_3$) -79.38 (3F) -81.0 (2F), -116.9 (2F). This salt was dissolved in 100 mL of methanol under nitrogen and treated with 79.95 mL of 0.9908 M aqueous lithium hydroxide. After stirring for 15 minutes, the solution was evaporated to dryness at 65-75°C under vacuum. The solid was dissolved in methanol, concentrated in vacuum and dried at 0.1 mm. The resulting solid was dissolved in 175 mL of ether and hexane was added slowly until a red oil precipitated leaving a colorless upper layer. The upper layer was decanted and evaporated giving 30.9 g of crude title salt. The salt was twice recrystallized from mixtures of ether and herane to give 29.7 g (60%) of the title product as a white powder. $^1H$ NMR (δ, $CD_3OD$) 7.20 (d, 2H), 7.60 (d, 2H); $^{19}F$ NMR (δ, $CD_3OD$) -79.02 (3F) -80.21 (2F), -115.5 (2F). Anal. Calcd. for $C_9H_4BrF_7LiNO_5S_2$: C, 22.06; H. 0.82; N, 2.86; F, 27.14; S.13.08; Br. 16.30; Li, 1.42. Found: C, 22.16; H, 0.83; N, 2.85; F, 25.66; S, 12.57; Br, 16.14; Li, 1.34.

EXAMPLE 6

Synthesis of Lithium N-(Trifluoromethanesulfonyl)-2-(4-ethenylphenoxy)tetrafluoroethanesulfonamide

A 1-L pressure vessel was charged under nitrogen with 73.5 g (0.15 mol) of lithium N-(trifluoromethanesulfonyl)-2-(4-bromophenoxy)tetrafluoroethanesulfonamide, 300 mL of acetonitrile, 1.15 g $Pd(OAc)_2$, 3.09 g of tri-o-tolylphosphine and 120 ml of triethylamine. The autoclave was closed, cooled, evacuated and charged with ethylene to 100 psi. The mixture was heated with stirring to 85°C for 14 hr, holding the gas pressure at 125 psi by venting or adding ethylene as needed. The mixture was cooled to room temperature and vented to atmospheric pressure. The autoclave contents were recovered using a mixture of acetonitrile and water to rinse. The mixture was treated with 6.3 g of lithium hydroxide monohydrate and 100 mL of water with vigorous stirring. Ether (300 mL) was added and the mixture was filtered through celite. A trace of 4-tert-butylcatechol was added to the filtrate which was concentrated to a solid. The residue was dissolved in ether. A small aqueous layer was separated and the ether was dried over anhydrous sodium sulfate. This solution was filtered and concentrated to an oil on a rotary evaporator. Methylene chloride (50 mL) was added and the mixture was filtered. Hexane was added to the cloud point and the mixture was filtered. The filtrate was concentrated under vacuum resulting in separation of an oil. Trituration of the oil with hexane caused crystallization. The crystals were collected and dried giving 53.1 g (81%) of the title product. A trace of 4-tert-butylcatechol was added to prevent polymerization. $^1H$ NMR (δ, acetone-d6) 5.25 (d, 1H), 5.80 (d, 1H), 6.80 (dd, 1H), 7.30 (d, 2H), 7.55 (d,

2H); $^{19}F$ NMR (δ, acetone-d6) -78.78 (3F) -79.77 (2F), -115.52 (2F). Anal. Calcd. for $C_{11}H_7F_7NO_5S_2Li{\bullet}2.4H_2O$: C, 27.49; H, 2.48; N, 2.91; F, 27.67; Li, 1.44; S, 13.34. Found: C, 27.48; H, 2.24, N, 3.03; F, 28.55; Li, 1.47; S, 15.26.

EXAMPLE 7

Synthesis and Homopolymerization of Lithium N-(Trifluoromethanesulfonyl)-2-(4-ethenylphenoxy) tetrafluoroethanesulfonimide

A 400 mL pressure vessel was swept with nitrogen and charged with 32.8 g (0.067 mol) of lithium N-(trifluoromethanesulfonyl)-2-(4-bromophenoxy)-tetrafluoro-ethanesulfonimide, 200 mL of acetonitrile, 80 mL of triethylamine, 0.47 g of palladium acetate and 1.32 g of tri-o-tolylphosphine. The vessel was closed, cool, evacuated and charged with ethylene to 100 psi. The vessel was heat to 85°C and the internal pressure was maintained at 120 psi with ethylene gas for 22 hr. The vessel was cooled to room temperature and vented. The vessel contents were rinsed out with a mixture of ether and water and treated with 2.82 g of lithium hydroxide in water. This mixture was filtered through Celite and the filtrate was concentrated under vacuum. The residue was dissolved in ether, filtered and concentrated under vacuum to 35.9 g of a paste. This material was dissolved in ether and treated with hexane to precipitate a dark oil. The solution was decanted and the oil was again extracted with ether and treated with hexane to precipitate a solid which was discarded. The combined ether/hexane solutions were concentrated and the residue was recrystallized from ether hexane to give 17.6 g of white solid in three crops. $^1H$ NMR (δ, $CD_3CN$) 5.3 (d, 1H), 5.8 (d, 1H), 6.78 (q, 1H), 7.25 (d, 2H), 7.50 (d, 2H); $^{19}F$ NMR (δ, $CD_3CN$) -78.9 (3F) -79.6 (2F), -115.5 (2F). Weaker peak in both the proton and fluorine NMR spectra suggested the presence of a small amount of the starting material. In an attempt at further purification, the product was dissolved in ether in the air and hexane was added to precipitate a gummy solid. It was recognized from the differing solubility characteristics and loss of olefinic resonances in the proton NMR spectrum that the product had polymerized. The solid was dissolved in 50 mL of deionized water and was dialyzed for several days against 2 X 2-L of deionized water in a 3500 MW cutoff dialysis tube. The solution in the tube was concentrated and dried at 100°C and 0.1 mm pressure to give 10.9 g of off-white solid polymer. $^1H$ NMR (δ, $CD_3OD$) 1.50 (broad, 3H), 6.5 and 6.9 (broad, 5H); $^{19}F$ NMR (δ, $CD_3OD$) -78.81 (3F) -79.47 (2F), -114.8 (2F). Anal. Calculated for $C_{11}H_7F_7S_2O_5NLi{\bullet}2H_2O$: C, 27.92; H, 2.34; N, 2.96; F, 28.10, Li, 1.47; S, 13.55. Found: C, 27.98; H, 2.37; N, 2.97; F, 26.65; Li, 1.40; S 13.84.

EXAMPLE 8

Synthesis of 4-CN-Ph-$OCF_2CFHOCF_2CF(CF_3)OCF_2CF_2SO_3Li$

A solution of 4-cyanophenol (7.15 g, 0.06 mol) in 80 mL of DMF was treated with 0.24 g (0.003 mol) of lithium tert-butoxide at room temperature. After stirring for 5 minutes, solid $CF_2{=}CFOCF_2CF(CF_3)OCF_2CF_2SO_3Li$ (28.4 g, 0.063 mol) was added in one portion resulting in an exotherm to 36°C. The resulting solution was stirred for 24 hr at room temperature. Concentrated hydrochloric acid (3 mL) was added and the mixture was concentrated on a rotary evaporator to a solid which was dried at 140-145°C and 0.05 mm giving 35.2 g of white solid. H NMR (δ, acetone-D6) 6.95 (d, 1H), 7.5 (d, 2H), 7.9 (d, 2H); $^{19}F$ NMR (δ, acetone-d6) -78 to -80 (5 F, $CF_3$ and $CF_2$), -81 to -89 (4F, $CF_2$'s), -117.3 (2F, $CF_2SO_3$), -144.2 (1F, tertiary F), -145.0 (1F, ddt, CHF).

EXAMPLE 9

Synthesis of

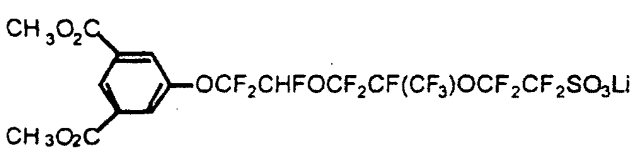

Dimethyl-5-hydroxyisophthalate (28.76 g, 0121 mol) was dissolved in 200 mL of anhydrous DMF under argon. Lithium tert-butoxide ((0.749 g) was added and the mixture was warmed to 40°C, then cooled to room temperature. Solid $CF_2{=}CFOCF_2CF(CF_3)OCF_2CF_2SO_3Li$ (51.3 g, 0.114 mol) was added and the solution was warmed to 40°C. A slight exotherm was noted. The solution was stirred for 2 hr at 40°C, then allowed to stir for 3 days at room temperature.

Hydrochloric acid (9.5 mL of 1.0 M) was added and the solution was concentrated on a rotary evaporator. The residue was dried at 145-150°C and 0.05 mm on a Kugelrohr. The dried solid was dissolved in 500 mL of ether. Hexane was added dripwise until a gummy precipitate formed. The mixture was filtered and the filtrate was concentrated and dried at 100°C and 0.1 mm giving 74.0 g of white solid. Anal. Calcd. for $C_{17}H_{10}F_{13}O_{10}SLi$: C, 30.92; H, 1.53; F, 37.41; S, 4.86; Li, 1.05. Found: C, 30.77; H, 1.77; F, 38.89; S, 4.69; Li 0.99.

EXAMPLE 10

Synthesis of Dimethyl 5-(1,1,2,2-tetrafluoro-2-bromoethoxy)isophthalate

A solution of 70.5 g of 95% potassium methoxide (0.956 mol) in 500 mL dry methanol was added to a suspension of 200.97 g (0.956 mol) dimethyl 5-hydroxyisophthalate in 400 mL of dry methanol cooled to 0-5°C. The mixture was allowed to warm to room temperature and decanted from a small amount of white solid. The methanol solution was concentrated on a rotary evaporator and the solid was dried at 150°C and 0. 1 mm to give 226.4 g. This salt was dissolved in 600 ml of dry DMSO and heated to 65°C. 1.2-Dibromotetrafluoroethane (259.8 g, 1 mole) was added dropwise resulting in an exotherm to 80°C. After addition was complete, the mixture was maintained at 75-85°C for 4 hr. It was cooled to room temperature and diluted to 2 L with ice water. The aqueous solution was decanted from a viscous gum and extracted with 200 mL methylene chloride. The methylene chloride extract was concentrated and the residue was combined with the viscous gum and washed with water. The organic material was taken up in methylene chloride, dried over anhydrous sodium sulfate and concentrated on a rotary evaporator. The residue was distilled in a Kugelrohr apparatus at 140°C and 0.2 mm giving 257.6 g of material which was 93% pure by glpc. Chromatography on silica gel, eluting with hexane and then 1 $\rightarrow$ 4% ethyl acetate in hexane gave the desired product in the first fractions. The combined fractions were concentrated on the rotary evaporator and the residue was distilled in a Kugelrohr at 125°C and 0.1 mm to give 239.6 g (62%) of the title product. $^1$H NMR (δ, $CDCl_3$) 3.98 (s, 6H), 8.05 (m, 2H), 8.60 (m, 1H); $^{19}$F NMR (δ, $CDCl_3$) -68.7 (2F), -86.4 (2F). Anal. Calcd. for $C_{12}H_9F_4BrO_5$: C, 37.04; H, 2.33; F, 19.53; Br, 20.54. Found: C, 36.95; H, 2.08; F, 19.34; Br, 20.57.

EXAMPLE 11

Synthesis of 3,5-di($CO_2CH_3$)-Ph-$OCF_2CF_2SO_2Cl$

A 5-L round bottom flask was charged with 109.2 g sodium bicarbonate, 600 mL of deionized water, 226.4 g of sodium dithionite and 300 mL of DMF. The mixture was heated to 65°C and dimethyl 5-(1,1,2,2-tetrafluoro-2-bromoethoxy)isophthalate (316.2 g, 0.81 mol) was added over 15 min. After addition was complete, the mixture was warmed to 80-85°C for 4 hr and then maintained at 50°C overnight. The mixture was cooled to room temperature and filtered. The solid was washed with ethyl acetate which was added to the filtrate. A lower aqueous layer was extracted with 2 X 100 mL methylene chloride and 2 X 100 mL ethyl acetate. All the organic layers were combined, washed with 3 X 50 mL brine, dried over anhydrous sodium sulfate and concentrated on a rotary evaporator. The residue was dried at 120°C and 0.1 mm to give a yellow-orange solid which was used in the next step without further purification. $^1$H NMR (δ, DMSO-d6) 3.95 (s, 6H), 7.97 (m, 2H), 8.40 (m, 1H); $^{19}$F NMR (δ, $CDCl_3$) -80.9 (2F), 131.3 (2F). This solid was dissolved in 1-L deionized water and 300 mL of CFC-113 was added. The flask was fitted with a dry ice condenser. Chlorine gas was bubbled into the mixture until an excess was present. A precipitate formed which was not completely soluble in the CFC-113 so 500 mL of methylene chloride were added. The excess chlorine was vented into a scrubber. the organic layer was separated and the aqueous layer was extracted with 3 X 250 mL methylene chloride. The combined organic layers were washed with 100 mL brine, dried over anhydrous sodium sulfate. concentrated on a rotary evaporator and distilled using a Kugelrohr at 150°C and 0.2 mm to give 277.3 g of faintly yellow solid. This material was recrystallized from CFC-113 to give, in three crops, 257.4 g (78 %) of white solid. $^1$H NMR (δ, $CDCl_3$) 3.95 (s, 6H), 8.10 (m, 2H), 8.70 (m, 1H); $^{19}$F NMR (δ, $CDCl_3$) -79.0 (2F), -108.0 (2F). Anal. Calcd. for $C_{12}H_9ClF_4O_7S$: C. 35.26; H, 2.22; Cl, 8.67; F. 18.59; S, 7.84. Found: C, 35.39; H, 2.05: Cl, 8.95; F, 18.29; S, 7.66.

EXAMPLE 12

Synthesis of 3,5-di($CO_2CH_3$)-Ph-$OCF_2CF_2SO_3Li$

To a suspension of 262 g (0.64 mol) 3,5-di($CO_2CH_3$)-Ph-$OCF_2CF_2SO_2Cl$ in 1-L of anhydrous methanol was added 52.1 g (0.71 mol) of anhydrous lithium carbonate. This mixture was heated to 40°C, then allowed to stir at room temperature for 96 hr. The solution was filtered and concentrated on the rotary evaporator. The solid was recrystallized from 6-L acetonitrile, collecting two crops. The combined solid was dried at 180°C and 0.1 mm to give 179.1 g (71%)

of product. $^{1}H$ NMR (δ, DMSO-d6) 3.95 (s, 6H), 7.95 (m, 2H), 8.40 (m, 1H); $^{19}F$ NMR (δ, $CDCl_3$) -80.9 (2F), 116.5 (2F). Anal. Calcd for $C_{12}H_9F_4SO_8Li$: C, 36.38; H, 2.29; F, 19.18; S, 8.24; Li, 1.75. Found: C, 36.29; H, 2.47; F, 19.08; S, 8.24; Li, 1.69.

EXAMPLE 13

Homopolymerization of Lithium 2-(4-ethenylphenoxy)tetrafluoroethanesulfonate

Lithium 2-(4-ethenylphenoxy)tetrafluoroethanesulfonate (12.24 g, 0.04 mol) was dissolved in 75 mL of deionized water. This solution was concentrated under vacuum to remove about 60 mL of water in order to eliminate any remaining traces of organic solvents complexed to the salt. Deionized water (40 mL) was added to the residue and this solution was deoxygenated by two freeze, evacuate, thaw cycles. Ammonium persulfate (0.018 g, 0.00008 mol) was added and the solution was again deoxygenated by freeze, evacuate and thaw cyles. The solution under argon was then heated in a 61-63°C oil bath for 26 hr. The solution was cooled to room temperature and transferred to 3500 MW cutoff dialysis tubing and dialyzed against three changes of 1-L of deionized water over several days. The aqueous solution in the dialysis tubing was concentrated on a rotary evaporator and the residue was dried at 100°C and 0.1 mm giving 9.33 g (76%) of faintly yellow solid polymer. H NMR (δ, $D_2O$) 1.50 (bs, 3H), 6.5 and 7.0 (bs, 5H); $^{19}F$ NMR (δ, $D_2O$) -82.02 (2F), -117.58 (2F). Mw, determined by light scattering in water containing 0.25% LiCl, was measured to be 156,000. Anal. Calcd. for $C_{10}H_7O_4F_4SLi{\bullet}1.67H_2O$: C, 35.73; H, 3.08; F, 22.61; LI, 2.06; S, 9.54. Found: C, 35.72; H, 3.17; F, 19.43, Li, 2.27; S, 10.03. A film of the polymer cast from water followed by air drying was light amber and self-supporting but somewhat brittle.

EXAMPLE 14

Copolymerization of Lithium 2-(4-ethenylphenoxy)tetrafluoroethanesulfonate and Styrene

A polymer tube was charged with 4.59 g (0.015 mol) of lithium 2-(4-ethenylphenoxy)tetrafluoroethanesulfonate, 8.0 mL of DMF, 8.84 g (0.085 mol) of freshly purified styrene and 0.05 g of benzoyl peroxide. The solution was frozen, evacuated, purged with argon and thawed several times and then heated under an argon atmosphere in a 60°C oil bath for 66 hr. After cooling to room temperature, the solid mass was dissolved in 50 mL of DMF and precipitated into excess ether. The ether was decanted and the gummy residue was washed with hexane and dried at 100°C and 0.5 mm giving 11.8 g (88%) of white copolymer. A clear, colorless film could be cast from a mixture of THF with 2% DMF. $^{1}H$ NMR (δ, DMF-D7) 1.65 and 2.0 (broad), 6.8 and 7.2 (broad); $^{19}F$ NMR (δ, DMF-d7) -80.9 (2F), -116.4 (2F); $^{13}C$ NMR (δ, DMF-d7) 118.3 and 114.09 ($CF_2$'s), 147.77 (aromatic C next to O), 146.05 ( aromatic C next to $CH_2$), 122.09 (aromatic C ortho to O), 128.7-128.2 (remaining aromatic C), 40.97 (CH) and 42-48 ($CH_2$). From integration of appropriate resonances in the F and H decoupled $^{13}C$ spectrum, calculate that the polymer contains 88% styrene and 12% of functionalized styrene. DSC showed a Tg at 135.8°C on second heat. Anal. Found: C, 71.52; H, 5.81; F, 8.76; Li, 0.72; S, 3.55.

EXAMPLE 15

Terpolymerization of Lithium 2-(4-ethenylphenoxy)tetrafluoroethanesulfonate with Styrene and Acrylonitrile

Styrene and acrylonitrile were passed through short columns of basic alumina and then distilled from calcium hydride immediately before using. A polymer tube was charged with 3.06 g (0.011 mol) of lithium 2-(4-ethenylphenoxy) tetrafluoroethane-sulfonate and 8 mL of DMF. The solution was placed under vacuum for 1 hr at room temperature to remove volatile impurities. Then 5.41 g (0.052 mol) styrene, 2.01 g (0.038 mol) acrylonitrile and 0.048 g of benzoyl peroxide were added. The solution was frozen, evacuated, purged with argon and thawed several times and then heated under an argon atmosphere in a 60°C oil bath for 22 hr. After cooling to room temperature, the solid mass was dissolved in DMF, filtered and poured slowly into excess ether. The sticky precipitate was dried under vacuum at 95°C to give 10.7 g of white polymer. $^{1}H$ NMR (d, THF-d8) 1.70-2.2 (broad). 6.8 and 7.05 (broad) and 2.0 (broad); $^{19}F$ NMR (d, THF-d8) -81.5 (2F), -117.2 (2F); IR 2237 $cm^{-1}$(CN); $^{13}C$ NMR (d, DMF-d7) 118.3 and 114.09 ($CF_2$'s), 122.08 (aromatic carbon ortho to O and CN from acrylonitrile), 147.77 (aromatic carbon adjacent to O), 146.0 to 140 (quaternary carbons), 128.4 to 127.1 (remaining aromatic carbons), 38.83 (aliphatic CH from styrenes) 28.5 to 26.6 (aliphatic CH from acrylonitrile) and 43 to 40 ($CH_2$). From integration of the carbon NMR spectrum, the polymer was calculated to contain 52.2 mole % styrene. 38.8 mole % acrylonitrile and 9.0% lithium 2-(4-ethenylphenoxy)tetrafluoroethanesulfonate. Anal. Found: C, 69.03; H, 6.00; N, 6.37; F, 6.59; S, 2.95; Li, 0.52.

EXAMPLE 16

Terpolymerization of Lithium 2-(4-ethenylphenoxy)tetrafluoroethanesulfonate with Styrene and Butyl Acrylate

The procedure of Example 15 was followed using 3.06 g (0.01 mol) of lithium 2-(4-ethenylphenoxy)tetrafluoroethanesulfonate, 6.55 g (0.063 mol) of styrene and 3.46 g (0.027 mol) of butyl acrylate. The crude product, dissolved in DMF, was precipitated by adding to excess ice water in a blender. After drying in a vacuum oven at 50°C, 7.10 g of polymer was obtained. $^1$H NMR (d, DMF-d7) 1.05 (broad) 1.3-2.6 (broad), 3.8 (broad), 6.95 and 7.40 (broad); $^{19}$F NMR (d, DMF-d7) -81.0 (2F), -116.6 (2F); $^{13}$C NMR (d, DMF-d7) 118.4 and 114.1 ($CF_2$'s), 122.2 (aromatic carbon ortho to O), 148.5 (aromatic carbon adjacent to O), 143.0 to 146.9 (quaternary carbons), 125.0 to 130.0 (remaining aromatic carbons), 38.85 (aliphatic CH from styrenes), 42 to 48 ($CH_2$ from styrenes), 175.9 (ester carbonyl), 64.06 ($OCH_2$), 14.06 ($CH^3$), 19.75 and 13.03 (remaining $CH_2$'s from ester). From integration of the carbon NMR spectrum, the polymer was calculated to contain 66.4 mole % styrene, 26.5 mole % butyl acrylate and 7.1% lithium 2-(4-ethenylphenoxy) tetrafluoroethanesulfonate.

EXAMPLE 17

Copolymerization of Lithium 2-(4-ethenylphenoxy)tetrafluoroethanesulfonate and Methyl Methacrylate

Methyl methacrylate was passed through a short column of basic alumina and distilled under vacuum into a receiver cooled in dry ice. A polymer tube was charged with 3.06 g (0.01 mol) of lithium 2-(4-ethenylphenoxy)tetrafluoroethanesulfonate, 9.0 g (0.09 mol) of freshly purified methyl methacrylate and 0.05 g of 2,4-dimethyl-2,2'-azobis(pentanenitrile) intiator (Vazo® 52). The solution was frozen, evacuated, purged with argon and thawed several times. The polymer tube was sealed and heated in a 50°C oil bath for 22 hr. After cooling to room temperature, the solid mass was dissolved in 250 mL of acetone with warming and filtered. The acetone was evaporated and the solid polymer was dried at 100°C and 0.05 mm giving 11.6 g of product. $^1$H NMR (δ, acetone-d6) 0.6-3.0 (m), 3.65 (s). 6.8-7.5 (m). From intergration of the appropriate peaks, the polymer contained 88 mole % methyl methacrylate and 12 mole % of lithium 2-(4-ethenylphenoxy)tetrafluoroethanesulfonate. $^{19}$F NMR (δ, acetone-d6) -80.9 (2F), -116.6 (2F). Anal. Found: C, 54.39; H, 6.77; F, 4.86; Li, 0.50; S, 2.62. A clear. flexible film of the polymer could be cast from acetone solution.

EXAMPLE 18

Copolymerization of Lithium 2-(4-ethenylphenoxy)tetrafluoroethanesulfonate and Acrylonitrile

Acrylonitrile was passed through a short column of basic alumina and vacuum distilled immediately before use. A 50 mL polymer tube was charged with 3.06 g (0.01 mole) of lithium 2-(4-ethenylphenoxy)tetrafluoroethanesulfonate, 10 mL DMF, 4.77 g (0.09 mole) acrylonitrile and 0.050 g of 2,4-dimethyl-2,2'-azobis(pentanenitrile) intiator (Vazo® 52). The vessel contents were deoxygenated by 4 freeze/evacuate/thaw cycles and the vessel was sealed. The contents were heated in an oil bath at 50°C for 22 hr resulting in formation of a white mass. The solid was dissolved in 50 mL DMF, filtered and precipitated into a large excess of ether. After drying at 100°C and 0.05 mm, 7.59 g (97%) of white polymer was isolated. $^1$H NMR (δ, DMF-d7) 2.30 (b), 3.32 (b), 7.3-7.5 (b) plus absorptions suggesting small amounts of DMF. By integration of the aromatic versus the aliphatic peaks, calculate the polymer contains 12 mole % lithium 2-(4-ethenylphenoxy)tetrafluoroethanesulfonate and 88 mole % acrylonitrile. Anal. Found: C, 54.72; H, 4.51; N, 15.55; F, 8.36; Li, 0.81; S, 4.04.

EXAMPLE 19

Copolymerization of Lithium 2-(4-ethenylphenoxy)tetrafluoroethanesulfonate and α-Methylene-γ-butyrolactone

A polymer tube was charged with 1.53 g (0.005 mol) of lithium 2-(4-ethenylphenoxy)tetrafluoroethanesulfonate, 4.4 g (0.045 mol) of α-methylene-γ-butyrolactone and 0.025 g of Vazo® 52. The salt dissolved nearly completely on stirring. DMF (100 uL) was added to give a completely homogeneous solution. The vessel contents were deoxygenated by 5 freeze/evacuate/thaw cycles and the solution was heated for 22 hr at 50°C under argon giving a solid yellow mass. This material was dissolved in 30 mL DMF with warming, filtered and precipitated into excess ether. Drying at 109°C and 0.1 mm gave 5.24 g (88%) of white polymer. $^1$H NMR (δ, DMF-d7) 225 (b), 2.5 (b), 7.3 - 7.5 (b). From the peak integrals, calculate the polymer composition as 11 mole % lithium 2-(4-ethenylphenoxy)tetrafluoroethanesulfonate. $^{19}$F NMR (δ, DMF-d7) -80.61 (2F), -116.31 (2F). DSC: Tg = 220°C. Anal. Found: C. 53.40; H, 5.53; F, 6.11, Li, 0.54; S, 2.75.

EXAMPLE 20

Copolymerization of Lithium 2-(4-ethenylphenoxy)tetrafluoroethanesulfonate with Poly(ethylene glycol) Ethyl Ether Methacrylate

In a glovebox, 1.21 g (.00395 mol) of lithium 2-(4-ethenylphenoxy)tetrafluoroethanesulfonate and 8.79 g (.0357 mol) of poly(ethylene glycol) ethyl ether methacrylate were combined in a Schlenk tube. Then 48 mg ($1.93 \times 10^{-4}$ mol) Vazo® 52 was added and stirred to dissolve. The flask was heated under argon at 40°C for 28 hours. The resultant polymer was soft and gummy, but insoluble and unpressable. Most likely some difunctional methacrylate was present and resulted in a crosslinked material. The polymer was dried at 70°C under vacuum and 9.7 g (97%) of material recovered. No films were obtained.

EXAMPLE 21

Copolymerization of Lithium 2-(4-ethenylphenoxy)tetrafluoroethanesulfonate with Butyl Acrylate

In a glovebox, 2.10 g (.0069 mol) of lithium 2-(4-ethenylphenoxy)tetrafluoroethanesulfonate and 8.0 g (.062 mol) of butyl acrylate were combined in a Schlenk tube with a stir bar. Approximately 10 mL of DMF was added, followed by 48 mg ($1.93 \times 10^{-4}$ mol) of Vazo® 52 and stirred to dissolve. The flask was placed under argon and heated at 40°C for 24 hours. The resultant polymer was dissolved in DMF, precipitated as an oil into hexane (3x). The polymer was then dissolved into acetone and precipitated into water (2x). The resultant polymer was dried under vacuum at 75°C, giving 1.6 g (16%) product. $^{19}F$ NMR (d-acetone) : -82.4 (2F), -118.0 (2F) ppm. TGA ($N_2$, 10°C/min): onset of decomposition at 250°C. Molecular weight (SEC, Zytel 101 standard, solvent HFIP + 0.01M sodium triflate): Mn = 186,800; Mw = 324,700; Mw/Mn = 1.74; Mn = 182,500; Mw = 316,800; Mw/Mn = 1.74.

EXAMPLE 22

Copolymerization of Lithium 2-(4-ethenylphenoxy)tetrafluoroethanesulfonate with Ethylhexyl Acrylate

In a glovebox, 1.56 g (.0051 mol) of lithium 2-(4-ethenylphenoxy)tetrafluoroethanesulfonate was dissolved in 20 mL DMF and added to 8.44 g (0.046 mol) of ethylhexyl acrylate in a Schlenk tube with a stir bar, followed by the addition of 48 mg ($1.93 \times 10^{-4}$ mol) of Vazo® 52. The initiator was stirred to dissolve. The flask was placed under argon and heated at 40°C for 24 hours. The resultant polymer was dissolved with additional THF and precipitated into water, followed by precipitations from THF as an oil into hexane (3x). The polymer was dried under vacuum at 65°C giving 3.2 g (32 %) product. Films could be melt pressed by placing the polymer between Teflon® press sheets, preheating 2 minutes at 150°C, pressing at 2000 lbs/in$^2$, and then cooling under pressure. The polymer picks up moisture rapidly from air and becomes a gum. Films could also be solution cast by dissolving 0.5 g of polymer in 15 mL of THF and casting the solution into a 5.5 cm diameter Teflon® petri dish. $^{19}F$ NMR (d-acetone) δ: -84.4 (2F), -120.0 (2F) ppm. $^{1}H$ NMR (d-THF) δ: 7.1 (b). 3.95 (b), 3.8 (b), 2.9, 2.3 (b), 1.8-2 (b), 1.5-1.7 (b), 1.4, 1.2 (b). 0.9 ppm. Ratio of aromatic styrene signal to a methyl signal from the ethylhexyl acrylate indicated approximately 10 mol % incorporation of lithium 2-(4-ethenylphenoxy)tetrafluoroethanesulfonate. $^{13}C$ NMR (d-THF) δ: 172.4, 127.4, 120.3, 40.1, 37.4, 28.9, 27.5, 22.7, 12.1, 8.2 ppm. TGA ($N_2$, 10°C/min): onset of decomposition at 250°C. DSC ($N_2$, -100 to 200°C, 10°C/min): no $T_m$ detected. Anal. Found: % C 65.62, 65.51; % H 9.48, 9.69; % F 3.82; % Li 0.34; % S 1.86. Molecular weight (SEC, Zytel 101 standard, solvent HFIP + 0.01M Na triflate): $M_n$ = 161,500, $M_w$ = 315,000, $M_w/M_n$ = 1.95; $M_n$ = 140,300, $M_w$ = 318,100, $M_w/M_n$ = 2.27

EXAMPLE 23

Copolymerization of Lithium 2-(4-ethenylphenoxy)tetrafluoroethanesulfonate with Butyl Acrylate and Carbonatoglycidal Methacrylate

The carbonatoglycidal methacrylate (1.23 g, .0066 mol), butyl acrylate (6.76 g, .0528 mol), and lithium 2-(4-ethenylphenoxy)tetrafluoroethanesulfonate (2.02 g, .0066 mol) were combined in a flask in a glovebox. Vazo® 52 (45 mg, $1.8 \times 10^{-4}$ mol) was added and stirred to dissolve. The flask was placed under argon and heated at 45°C for 24 hour. The resultant polymer was insoluble in a variety of solvents and could not be pressed into a film. The polymer was dried under vacuum at 65°C to give 9.4 g (94%) of polymer.

EXAMPLE 24

Copolymerization of Lithium 2-(4-ethenylphenoxy)tetrafluoroethanesulfonate with Butyl Acrylate

In a glovebox, 2.10 g (.0069 mol) of lithium 2-(4-ethenylphenoxy)-tetrafluoroethanesulfonate and 7.9 g (.062 mol) of butyl acrylate were combined in a Schlenk tube with a stir bar, followed by the addition of 45 mg (1.81 x $10^{-4}$ mol) of Vazo® 52 and stirred to dissolve. The flask was placed under argon and heated at 40°C for 24 hours, and 45°C for 24 hours. The resultant polymer was dissolved in THF, precipitated into water (2x) and hexane (2x) as an oil. The polymer was dried under vacuum giving 0.2 g (2% yield) polymer. $^{13}C$ NMR (d-THF) δ: 175.1. 67.6, 64.9, 42.6, 31.8, 25.8, 20.2, 14.3 ppm. TGA ($N_2$, 10°C/min): onset of decomposition at 250°C.

EXAMPLE 25

Copolymerization of Lithium 2-(4-ethenylphenoxy)tetrafluoroethanesulfonate and Butyl Acrylate

In a glovebox, 3.74 g (.0122 mol) of lithium 2-(4-ethenylphenoxy)tetrafluoroethanesulfonate and 6.26 g (.0489 mol) of butyl acrylate were combined in a Schlenk tube with a stir bar, followed by the addition of 45 mg (1.81 x $10^{-4}$ mol) of Vazo® 52 and stirred to dissolve. The flask was placed under argon and heated at 45°C for 24 hours. The resultant polymer was dissolved in acetone/THF and precipitated into water. The polymer was then precipitated from acetone/THF into hexane (2x). The polymer was dried to obtain 4.8 g (48%). Films could be cast by dissolving 0.5 g of polymer in THF and casting the solution into a 5.5 cm diameter Teflon® petri dish. $^{19}F$ NMR (d-acetone) δ: -82.4 (2F), -118.1 (2F) ppm. $^{13}C$ NMR (d-acetone) δ: 175.5, 130.3, 123.1, 65.3, 42.8, 36.6 (b), 31.9, 20.3, 14.5 ppm. $^{1}H$ NMR (d-acetone) δ: 7.1 (b), 4.1, 3.9 (b), 3.2, 3.1, 2.4, 1.9 - 1.2, 0.9 ppm. Anal. Found: % C 54.34, 54.29; % H 6.95, 6.91; % F 9.46, 9.31; % Li 0.71, 0.80; % S 4.40, 4.47 TGA ($N_2$, 10°C/min): onset of decomposition at 275°C. DSC ($N_2$, -100 to 225°C, 10°C/min): no $T_m$ detected. Molecular weight (SEC, Zytel 101 standard, HFIP + .01 M Na inflate): $M_n$ = 432,000 $M_w$ = 1,271,000 $M_w/M_n$ = 2.94 $M_n$ = 980,700, $M_w$ = 1,415,000, $M_w/M_n$ = 1.44.

EXAMPLE 26

Copolymerization of Lithium 2-(4-ethenylphenoxy)tetrafluoroethanesulfonate and Methyl Acrylate

In a glovebox, 2.8 g (.0091 mol) of lithium 2-(4-ethenylphenoxy)tetrafluoroethanesulfonate and 7.2 g (.0827 mol) of methyl acrylate were combined in a Schlenk flask with a stir bar, followed by the addition of 35 mg ( 1.41 x $10^{-4}$ mol) of Vazo® 52 and stirred to dissolve. The flask was placed under argon and heated at 35°C for 48 hours, followed by 65°C for 24 hours. The polymerization phase separated. Portions of the polymer were harder to dissolve than others, and some insolubles were present. The resultant polymer was dissolved in DMF/acetone and precipitated into hexane (2x). The polymer was then dissolved into DMF and dialyzed in dialysis tubing (MWCO = 3500) against water for 10 days, followed by collecting the polymer, dissolving it in DMF, and precipitating into water. The polymer was dried under vacuum to give 3.14 g (31.4%) of product. $^{19}F$ NMR (d-DMF) δ: -81.9 (2F), -117.6 (2F) ppm.

EXAMPLE 27

Copolymerization of Lithium 2-(4-ethenylphenoxy)tetrafluoroethanesulfonate and Methyl Acrylate

In a glovebox. 2.8 g (.0091 mol) of lithium 2-(4-ethenylphenoxy)tetrafluoroethanesulfonate, 7.2 g (.0827 mol) of methyl acrylate and 20 mL of DMF were combined in a Schlenk tube with a stir bar, followed by the addition of 35 mg (1.41 x $10^{-4}$ mol) of Vazo® 52. The flask contents were stirred to dissolve. The flask was placed under argon and heated at 35°C for 48 hours. The resultant polymer was dissolved in DMF/acetone and precipitated into water (3x). The polymer was then dissolved into acetone and precipitated into hexane. The final polymer was dried under vacuum at 70°C to give 4.85 g (48.5%) product. Films were cast by dissolving 0.54 g of polymer in acetone and casting the solution into a 5.5 cm diameter Teflon® petri dish. $^{19}F$ NMR (d-acetone) δ: -82.4 (2F), -118.1 (2F) ppm. $^{13}C$ NMR (d-acetone) δ: 174.9, 129.2, 12.1, 51.2, 41.3, 35.1 (b) ppm. $^{1}H$ NMR (d-acetone) δ: 7.2 (b), 3.6, 2.9, 2.8, 2.1, 3.5, 2.4 (b), 1.9 (b), 1.7 (b), 1.7-1.5 (b) ppm. Proton NMR indicated 4 mole percent ionomeric groups based on integration of aromatic signal and methyl group signal of the methyl acrylate. Molecular weight (SEC, Zytel 101 standard, HFIP + .01 M Na triflate): $M_n$ = 77,200, $M_w$ = 216,100, $M_w/M_n$ = 2.8; $M_n$ = 56,500, $M_w$ = 203,600, $M_w/M_n$ = 3.6. Anal. Found: % C 53.16, 53.13, 52.78; % H 6.77, 6.79, 6.36; % F 3.24, 3.07, 4.81; % Li 0.28, 0.47, 0.38; % S 1.91, 1.98, 2.39. Elemental analysis indicated 5-6 mole percent lithium 2-(4-ethenylphenoxy)tetrafluoroethane-sulfonate present.

EXAMPLE 28

Copolymerization and Crosslinking of Lithium 2-(4-ethenylphenoxy)tetrafluoroethanesulfonate, Methyl Methacrylate, and Trimethylolpropane Triacrylate

Crosslinked films of methylmethacrylate and lithium 2-(4-ethenylphenoxy)-tetrafluoroethanesulfonate were made in the following manner. Methyl methacrylate (7.5 g, 7.5 x $10^{-2}$ mole) and sulfonate monomer (2.5 g, 8.2 x $10^{-3}$ mol) were premixed.

Film A: A 3.33 g aliquot of the monomer mixture was placed in a vial, 0.041 g (.00014 mol) of triacrylate and 20-24 mg of Vazo® 52 initiator were added and stirred until dissolved. The mixture was poured into a small Teflon® casting dish and heated on a hot plate under a nitrogen atmosphere.

Film B: A 3.33 g aliquot of the monomer mixture was added to 0.16 g (.00054 mol) of triacrylate and 20-24 mg of Vazo® 52 initiator in a vial and stirred until dissolved. The mixture was poured into a small Teflon® casting dish and heated on a hot plate under a nitrogen atmosphere.

Film C: A 3.22 g aliquot of the monomer mixture was added to 0.41 g (.0014 mol) of triacrylate and 20-24 mg of Vazo® 52 initiator in a vial and stirred until dissolved. The mixture was poured into a small Teflon® casting dish and heated on a hot plate under a nitrogen atmosphere.

After 30 minutes, the temperature of the hot plate had risen to 49.6°C and it appeared that the polymerization was occurring too quickly. Films B and C were forming bubbles and cracks. The heat was reduced and no further bubbling or cracking was observed. The films were heated for 4 hours, left at room temperature overnight, then heated at 45°C for 3 hours. The resultant films swelled in acetone but did not dissolve and could not be melt pressed. The films were dried under vacuum at 70°C. TGA ($N_2$, 10 °C/min): onset of decomposition at 260°C (A and B), 300°C (C).

EXAMPLE 29

Copolymerization and Crosslinking of Lithium 2-(4-ethenylphenoxy)tetrafluoroethanesulfonate, Poly(ethylene glycol) Ethyl Ether Methacrylate, and Poly(ethylene glycol) Diacrylate\

Crosslinked films of poly(ethylene glycol) ethyl ether methacrylate and lithium 2-(4-ethenylphenoxy)tetrafluoroethanesulfonate were made in the following manner. Poly(ethylene glycol) ethyl ether methacrylate (8.79 g, 3.6 x $10^{-2}$ mol) and the sulfonate monomer (1.21 g, 4.0 x $10^{-3}$ mol) were premixed.

Film A: A 3.33 g aliquot of the monomer mixture was placed in a vial with .038 g (6.6 x $10^{-5}$ mol) of poly (ethylene glycol) diacrylate and 16 mg (6.45 x $10^{-5}$ mol) of Vazo® 52 and stirred to dissolve. The mixture was poured into a small Teflon® casting dish and heated between 35-40°C on a hot plate under a nitrogen atmosphere for five hours.

Film B: A 3.33 g aliquot of the monomer mixture was placed in a vial with .076 g (1.32 x $10^{-4}$ mol) poly(ethylene glycol) diacrylate and 16 mg (6.45 x $10^{-5}$ mol) of Vazo® 52 and stirred to dissolve. The mixture was poured into a small Teflon® casting dish and heated between 35-40°C on a hot plate under a nitrogen atmosphere for five hours.

Film C: A 3.15 g aliquot of the monomer mixture was placed in a vial with .15 g (2.61 x $10^{-4}$ mol) poly(ethylene glycol) diacrylate and 16 mg (6.45 x $10^{-5}$ mol) of Vazo® 52 and stirred to dissolve. The mixture was poured into a small Teflon® casting dish and heated between 35-40°C on a hot plate under a nitrogen atmosphere for five hours.

The films were left at room temperature overnight and then heated at 37°C for 6 hours. Some gelling of the films upon heating was observed. The films were insoluble, soft, and flexible. The films were dried at 75°C under vacuum. Acetone used to extract small portions of the thinner films cast onto the plate was sent for $^{13}C$ NMR and showed no monomer signal. Acetone used to extract the thicker films from the casting dishes showed some residual monomer. TGA ($N_2$, 10°C/min): onset of decomposition 225°C (A, B) and 200°C (C). DSC ($N_2$, -100 to 200°C, 10°C/min): no $T_m$ detected. Anal. Found: Film A: % C 56.49; % H 8.17; % F 2.87; % S 0.74; ash 1.74; % Li 0.22. Film B: %C 56.45; %H 8.16; %F 2.33; %S 0.50; ash 1.83; % Li 0.23. Film C: % C 56.79; % H 8.24; % F 2.44; % S 0.57; ash 1.83; % Li 0.23.

EXAMPLE 30

Copolymerization and Crosslinking of Lithium 2-(4-ethenylphenoxy)tetrafluoroethanesulfonate, Butyl Acrylate and Carbonatoglycidal Methacrylate

In a glovebox, 1.23 g (.0066 mol) of carbonatoglycidal methacrylate, 6.76 g (.0528 mol) of butyl acrylate and 2.02 g (.0066 mol) of lithium 2-(4-ethenylphenoxy)tetrafluoroethanesulfonate were combined in a vial. Vazo® 52 (40 mg, 1.61 x $10^{-4}$ mol) was added and stirred to dissolve. The solution was pipetted into two small Teflon® petri dishes to just cover the bottom. The remaining solution was pipetted into squares on a Teflon®-coated piece of glass. The

solutions were warmed at 35°C on a slide heater in the glovebox for 2 hours, left at room temperature overnight, heated at 35°C for 8 hours, cooled to room temperature overnight, and then heated at 42°C for 8 hours. The hygroscopic films are brittle when dry, but handleable when wet. $^{19}F$ NMR (d-acetone) δ: -82.7 (2F), -118.5 (2F) ppm. TGA ($N_2$, 10°C/min): onset of decomposition at 200°C. DSC ($N_2$, -100 to 175°C, 10°C/min): no $T_m$ detected. Anal. Found: % C 50.64, 50.49, 50.66; % H 5.62, 5.58, 5.61; % F 9.84, 9.43, 9.24; % Li 0.38, 0.90, 0.84; % S 4.38, 3.83, 4.09; % N, 0.22, 0.18.

EXAMPLE 31

Copolymerization and Crosslinking of Lithium 2-(4-ethenylphenoxy)tetrafluoroethanesulfonate and Poly(ethylene glycol) Ethyl Ether Methacrylate

In a glovebox, 8.79 g (.0357 mol) of poly(ethylene glycol) ethyl ether methacrylate, 1.21 g (.00395 mol) of lithium 2-(4-ethenylphenoxy)tetrafluoroethanesulfonate, and 45 mg (1.81 x $10^{-4}$ mol) Vazo® 52 were combined. The solution was pipetted into 2 small Teflon® petri dishes to just cover the bottom, and the rest was pipetted into squares on a piece of Teflon®-coated glass. The films were warmed at 35°C on a slide warmer for 4 hr., left at room temperature overnight, and then warmed at 35°C for 8 hrs. The films phase separated upon polymerization to give flexible films that were glassy looking with grainy inclusions. Both phases were insoluble in THF. TGA ($N_2$, 10°C/min): onset of decomposition at 225°C. DSC ($N_2$, -100 to 175°C, 10°C/min): no $T_m$ detected. Anal. Found: % C 56.58, 56.33; % H 8.34. 8.33; % F 2.78, 2.63; % S 1.27, 1.30; % Li 0.23, 0.23. Elemental analysis indicated approximately 8 mole percent of monomer **1** incorporated.

EXAMPLE 32

Copolymerization and Crosslinking of Lithium 2-(4-ethenylphenoxy)tetrafluoroethanesulfonate and Poly(ethylene glycol) Ethyl Ether Methacrylate

The poly(ethylene glycol) ethyl ether methacrylate (7.63 g, .031 mol) and lithium 2-(4-ethenylphenoxy) tetrafluoroethanesulfonate (2.37 g, .0077 mol) were combined with 45 mg (1.81 x $10^{-4}$ mol) of Vazo® 52 in a vial in the glovebox The solution was pipetted into 2 small Teflon® petri dishes to just cover the bottom, and the rest was pipetted into squares on a piece of Teflon®-coated glass. The films were warmed at 35°C on a slide warmer for 4 hr., left at room temperature overnight, and then warmed at 35°C for 8 hrs. Upon polymerization the films phase separated. The flexible films appeared glassy with grainy inclusions. Both phases were insoluble in THF. TGA ($N_2$, 10°C/min): onset of decomposition at 225°C. DSC ($N_2$, -100 to 200°C, 10°C/min): no $T_m$ detected. Anal. Found: % C 53.85, 53.92; % H 7.55, 7.51; % F 5.00, 5.20; % S 2.36, 2.21; % Li 0.47, 0.46.

EXAMPLE 33

Copolymerization and Crosslinking of Lithium 2-(4-ethenylphenoxy)-tetrafluoroethanesulfonate and Poly(ethylene glycol) Ethyl Ether Methacrylate

In a glovebox, 8.79 g (.0357 mol) of poly(ethylene glycol) ethyl ether methacrylate and 1.21 g (.00395 mol) of lithium 2-(4-ethenylphenoxy)-tetrafluoroethanesulfonate were combined with 45 mg (1.81 x $10^{-4}$ mol) of Vazo® 52. The solution was pipetted into 2 small Teflon® petri dishes to just cover the bottom, and the rest was pipetted into squares on a piece of Teflon®-coated glass. The films were warmed at 35°C on a slide warmer for 5 hr., left at room temperature overnight, and then warmed at 45°C for 8 hrs. Some of the films phase separated while others remained homogeneous.

EXAMPLE 34

Copolymerization of Lithium N-(Trifluoromethanesulfonyl)-2-(4-ethenylphenoxy)tetrafluoroethanesulfonimide and Methyl Methacrylate

The procedure of Example 17 was followed using 4.36 g of lithium N-(trifluoromethanesulfonyl)-2-(4-ethenylphenoxy)tetrafluoroethanesulfonimide in place of the lithium 2-(4-ethenylphenoxy)tetrafluoroethanesulfonate giving 12.4 g of polymer. $^{1}H$ NMR (δ, acetone-D6) 0.6-3.1 (m), 3.65 (s), 6.9-7.7 (m). From intergration of the appropriate peaks, the polymer contains 88 mole % methyl methacrylate and 12 mole % of the sulfonimide monomer. $^{19}F$ NMR (δ, acetone-d6) -78.8 (s, 3F), -79.7 (m, 2F), -115.5 (s, 2F). Anal. Found: C, 49.77; H, 6.06, N. 1.03; F. 9.93; S, 5.08; Li, 0.50.

EXAMPLE 35

Copolymerization of Lithium N-(Trifluoromethanesulfonyl)-2-(4-ethenylphenoxy)tetrafluoroethanesulfonimide and Styrene

The procedure of Example 14 was followed using 4.37 g (0.01 mol) of lithium N-(trifluoromethanesulfonyl)-2-(4-ethenylphenoxy)tetrafluoroethanesulfonimide, 9.36 g (0.09 mol) of styrene, 8.0 mL of DMF and 0.048 g of benzoyl peroxide. Polymerization was conducted for 96 hr. The solid mass was dissolved in additional DMF and filtered. The polymer was precipitated into 1 L of ice water, filtered and dried at 96°C and 0.05 mm giving 8.38 g. Anal. Found: C, 70.31; H, 6.07; N, 1.75; F, 8.69; S, 4.63; Li, 0.44.

EXAMPLE 36

Copolymerization of Lithium N-(Trifluoromethanesulfonyl)-2-(4-ethenylphenoxy)tetrafluoroethanesulfonimide and Methyl Acrylate

This was an attempt to make a blockier polymer by letting methyl acrylate polymerize to viscous material and then adding the ionomeric monomer. In a glovebox, 7.2 g (.0827 mol) of methyl acrylate was dissolved in 10 mL DMF and 35 mg ($1.41x10^{-4}$ mol) of Vazo® 52 initiator was added. The flask was heated at 35°C. After one hour when the solution became very viscous, 10 mL of additional DMF was added along with 2.8 g (.0064 mol) of lithium N-(trifluoromethanesulfonyl)-2-(4-ethenylphenoxy)tetrafluoroethanesulfonimide in 10 mL of DMF. Heating was resumed at 35°C for 48 hr, 45°C for 24 hr, and 65°C for 24 hr. The resultant polymer was dissolved in DMF/acetone and precipitated into hexane (2x). The polymer was then dissolved into DMF and precipitated into water (2x). The polymer was dried under vacuum at 75°C to yield 5.6 g (56%) material. Films could be obtained by dissolving 0.5 g of the polymer in DMF and casting the solution into a 5.5 cm Teflon® petri dish.. $^{19}F$ NMR (d-DMF) δ: -79.8 (3F), -80.7 (2F), -116.5 (2F) ppm. $^{13}C$ NMR ($CDCl_3$) δ: 174.8 (C=O), 51.7 ($OCH_3$), 41.2 ($CH_2\underline{C}H$), 35.3 ($\underline{C}H_2CH$). Only methyl acrylate signals detected in the $^{13}C$ NMR. $^1H$ NMR ($CDCl_3$) δ: 7.3, 3.6, 2.9, 2.8, 2.3, 1.9, 1.7, 1.5 (b) ppm. Ratio of aromatic to $OCH_3$ (MA) peak integration indicated .6 mole percent of ionomeric groups. TGA ($N_2$, 10°C/min): onset of decomposition at 300°C. DSC ($N_2$, -100 to 200°C, 10°C/min): no $T_m$ detected, $T_g$ = 12°C (1st heat), 17°C (2nd heat). Anal. Found: % C 54.24, 54.35; % H 6.89, 6.62; % N < 0.1, < 0.1; % F 1.02, 1.27; % Li 0.042, 0.058; % S 0.84, 0.83. Molecular weight (SEC, Zytel 101 standard, HFIP + 0.01 M sodium triflate): Mn = 124,100; Mw = 370,300; Mw/Mn = 2.98; Mn = 134,400; Mw = 380,300; Mw/Mn = 2.83.

EXAMPLE 37

Copolymerization of Lithium N-(Trifluoromethanesulfonyl)-2-(4-ethenylphenoxy)tetrafluoroethanesulfonimide with Butyl Acrylate

In a glovebox, 2.75 g (.0063 mol) of lithium N-(trifluoromethanesulfonyl)-2-(4-ethenylphenoxy)tetrafluoroethanesulfonimide and 7.25 g (0.057 mol) of butyl acrylate were combined in a Schlenk tube with a stir bar, followed by the addition of 45 mg ($1.81 \times 10^{-4}$ mol) Vazo® 52 and stirred to dissolve. The flask was placed under argon and heated at 40°C for 24 hours, followed by 45°C for 24 hr. The resultant polymer was dissolved in THF, precipitated into water (2x). The polymer was then dissolved and precipitated into hexane (2x). The polymer was dried under vacuum to give 2.4 g (24%) polymer. Films were cast by dissolving 0.5 g of polymer in 1:1 THF/acetone and casting the solution into a 5.5 cm diameter Teflon® petri dish. A small amount of gelled material was present which did not dissolve. $^{19}F$ NMR (d-THF) δ: -80.4 (2F), -81.4 (2F), - 117.2 (2F) ppm. $^{13}C$ NMR (d-THF) δ: 175.1, 130.1, 122.7, 64.9, 42.6, 36.1, 31.8,20.2, 14.3 ppm. $^1H$ NMR (d-THF) δ: 7.1 (b), 4.0, 3.1, 2.0 - 3.0 (b), 1.1-1.8 (b), 0.8 ppm. The ratio of aromatic to methyl peak of butyl acrylate indicates approximately 15 mol % ionomeric monomer incorporated. TGA ($N_2$, 10°C/min): onset of decomposition at 275°C. DSC ($N_2$, -100 to 250°C, 10°C/min): no $T_m$ detected. Anal. Found: % C 52.49, 52.24; % H 6.90, 6.84; % N 1.19, 1.19; % F 10.02, 9.83; % S 4.82, 4.93; % Li 0.48, 0.51. Elemental analysis indicated 12 mole percent incorporation of the ionic monomer. Molecular weight (SEC, Zytel 101 standard, HFIP + 0.01 M Na inflate): $M_n$ = 250,200, $M_w$ = 1,024, 000, $M_w/M_n$ = 4.09; $M_n$ = 203,700, $M_w$ = 893,100, $M_w/M_n$ = 4.38

EXAMPLE 38

Copolymerization and Crosslinking of Lithium N-(Trifluoromethanesulfonyl)-2-(4-ethenylphenoxy) tetrafluoroethanesulfonimide and Poly(ethylene glycol) Ethyl Ether Methacrylate

In a glovebox, 1.64 g (.00376 mol) of lithium N-(trifluoromethanesulfonyl)-2-(4-ethenylphenoxy)tetrafluoroethanesulfonimide and 8.36 g (.02296 mol) of PEEGMA were combined in a vial with 5 mL of DMF. The Vazo® 52 (48 mg, 6.45 x $10^{-5}$ mol) was added and stirred until dissolved. A portion of the solution was used to coat the bottom of a 5.5 cm Teflon® petri dish. The dish was warmed on a slide heater in the glovebox at 39°C for 6 hrs, allowed to sit at room temperature overnight, followed by heating at 40°C for 8 hrs. Films were soft and flexible. TGA ($N_2$, 10°C/min): onset of decomposition at 225°C. DSC ($N_2$, 10°C/min): no $T_m$ detected. Anal. Found: % C 54.39; % H 7.57; % N 0.57; % F 3.87; % S 1.22; % Li 0.21; ash 1.64. Elemental analysis indicated approximately 9 mole % incorporation of the ionic monomer.

EXAMPLE 39

Copolymerization and Crosslinking of Lithium N-(Trifluoromethanesulfonyl)-2-(4-ethenylphenoxy) tetrafluoroethanesulfonimide, Poly(ethylene glycol) Ethyl Ether Methacrylate and Poly(ethylene glycol) Diacrylate

In a glovebox, 8.36 g (.03396 mol) of poly(ethylene glycol) ethyl ether methacrylate, 1.64 g (.00376 mol) of lithium N-(trifluoromethanesulfonyl)-2-(4-ethenylphenoxy)tetrafluoroethanesulfonimide, and 5 mL of DMF were combined in a vial and stirred to mix. Three films with different amounts of crosslinker were made as described below:

Film 1: 4.33 g (3.33 g monomers, 1 g DMF) of the monomer mixture were placed in a vial with .036 g (6.26 x $10^{-5}$ mole) diacrylate. Vazo® 52 (16 mg, 6.45 x $10^{-5}$ mol) was added and stirred to dissolve. Films were pipetted onto a Teflon®-coated piece of glass and warmed on a slide heater at 39°C.

Film 2: 4.33 g (3.33 g monomers, 1 g DMF) of the monomer mixture were placed in a vial with .071 g (1.24 x $10^{-4}$ mole) diacrylate and 16 mg (6.45 x $10^{-5}$ mole) of Vazo® 52 and stirred to mix. Films were pipetted onto a Teflon®-coated piece of glass and warmed on a slide heater at 39°C.

Film 3: Approximately 3.8 g of the monomer/DMF mixture, 0.14 g (2.44 x $10^{-4}$ mole) of diacrylate and 16 mg (6.45 x $10^{-5}$ mole) of Vazo® 52 were placed in a vial and stirred to mix. Films were cast as above and warmed on a slide heater at 39°C.

Films were heated for 6 hours at 39°C, cooled to room temperature overnight, then heated 8 hours at 40°C. Flexible films were formed. TGA ($N_2$, 10°C/min): onset of decomposition at 200C for all three films. DSC ($N_2$, -100 to 200°C, 10°C/min): no $T_m$ detected. Anal. Found. Film 1: % C 54.29; % H 7.72; % N 0.55; % F 4.02; % S 1.29; Film 2: % C 54.20; % H 7.68; % N 0.57; % F 4.40; % S 1.29; Film 3: % C 54.46; % H 7.75; % N 0.57; % F 3.84; % S 1.06. Elemental analysis indicated approximately 9 mole percent of monomer 2 incorporated.

EXAMPLE 40

Copolymerization and Crosslinking of Lithium N-(Trifluoromethanesulfonyl)-2-(4-ethenylphenoxy) tetrafluoroethanesulfonimide, Poly(ethylene glycol) Ethyl Ether Methacrylate, and Poly(ethylene glycol)Diacrylate

Film A: In a glovebox. the PEGEEMA (2.75 g, 1.12 x $10^{-2}$ mol), diacrylate (0.036 g, 6.26 x $10^{-5}$ mole, and lithium N-(trifluoromethanesulfonyl)-2-(4-ethenylphenoxy)tetrafluoroethanesulfonimide (0.55 g, 1.26 x $10^{-3}$ mol) were combined with 16 mg (6.45 x $10^{-5}$ mol) of Vazo® 52. The film was cast into a small Teflon® petri dish and heated at 40°C for 6 hours on a slide warmer.

Film B: In a glovebox, the PEGEEMA (2.76 g, 1.12 x $10^{-2}$ mol), diacrylate (.018 g, 3.13 x $10^{-5}$ mole, and lithium N-(trifluoromethanesulfonyl)-2-(4-ethenylphenoxy)tetrafluoroethanesulfonimide (0.55 g, 1.26 x $10^{-3}$ mol) were combined with 16 mg (6.45 x $10^{-5}$ mol) of Vazo® 52. The film was cast into a small Teflon® petri dish and heated at 40°C for 6 hours on a slide warmer.

Film C: In a glovebox, the PEGEEMA (2.77 g, 1.13 x $10^{-2}$ mol), diacrylate (0.009 g, 1.57 x $10^{-5}$ mole, and lithium N-(trifluoromethanesulfonyl)-2-(4-ethenylphenoxy)tetrafluoroethanesulfonimide (0.55 g, 1.26 x $10^{-3}$ mol) were combined with 16 mg (6.45 x $10^{-5}$ mol) of Vazo® 52. The film was cast into a small Teflon® petri dish and heated at 40°C for 6 hours on a slide warmer

Film D: The PEGEEMA (2.72 g, 1.10 x$10^{-2}$ mol), diacrylate (.071 g, 1.24 x $10^{-4}$ mole, and lithium N-(trifluoromethanesulfonyl)-2-(4-ethenylphenoxy)tetrafluoroethanesulfonimide (0.54 g, 1.23 x $10^{-3}$ mol) were combined with 16 mg (6.45 x $10^{-5}$ mol) of Vazo® 52. The film was cast into a small Teflon® petri dish and heated on a slide warmer at 40°C for 6 hours, left at room temperature overnight, and then heated at 50°C for 8 hr.

The films were dried at 85°C under vacuum. TGA ($N_2$, 10°C/min): onset of decomposition at 250°C (A, B, D) and 230°C (C). DSC ($N_2$, -100 to 200°C, 10°C/min): no $T_m$ detected. Anal. Found: Film A: % C 53.99; % H 7.97; % N 0.58; % F 4.70; % S 2.33; % Li 0.22; Film B: % C 54.11; % H 8.20; % N 0.58; % F 4.67; % S 2.82; % Li 0.21; Film C: % C 54.42; % H 8.29, % N 0.54, % F 4.53, % S 2.59, % Li 0.22; Film D: % C 54.17, % H 7.96, % N 0.55; % F 4.3;, % S 2.36; % Li 0.19. The elemental analysis of films extracted and then dried were close to the analyses of unextracted films and the calculated values (10 mole percent lithium N-(trifluoromethanesulfonyl)-2-(4-ethenylphenoxy)tetrafluoroethane-sulfonimide incorporated).

EXAMPLE 41

Copolymerization and Crosslinking of Lithium N-(Trifluoromethanesulfonyl)-2-(4-ethenylphenoxy) tetrafluoroethanesulfonimide, Methyl Acrylate, and Diallyldiglycol Carbonate

For each film, the following was done. The acrylate, lithium N-(trifluoromethanesulfonyl)-2-(4-ethenylphenoxy) tetrafluoroethanesulfonimide, and carbonate monomers were placed in a vial with 1 mL of DMF and a stir bar. The initiator was added and stirred to dissolve. The solution was pipetted into a Tetlon® petri dish and into squares on a piece of Teflon®-coated glass. The films were heated on a slide warmer at 40°C for several hours, left at room temperature overnight, and then heated at 45°C for 8 hrs. The amount of the monomers and initiator for each film are given below.

Film A: methyl acrylate (3.13 g, .036 mol), lithium N-(trifluoromethanesulfonyl)-2-(4-ethenylphenoxy) tetrafluoroethanesulfonimide (1.76 g, .00403 mol), diallyldiglycol carbonate (0.111 g, 4.05 x $10^{-4}$ mol), Vazo® 52 (15 mg, 6.04 x $10^{-5}$ mol).

Film B: methyl acrylate (3.17 g, .036 mol), lithium N-(trifluoromethanesulfonyl)-2-(4-ethenylphenoxy) tetrafluoroethanesulfonimide (1.78 g, .00407 mol), diallyldiglycol carbonate (0.0557 g, 2.03 x $10^{-4}$ mol), Vazo® 52 (15 mg, 6.04 x $10^{-5}$ mol).

Film C: methyl acrylate (3.18 g, .0365 mol), lithium N-(trifluoromethanesulfonyl)-2-(4-ethenylphenoxy) tetrafluoroethanesulfonimide (1.78 g, .00407 mol), diallyldiglycol carbonate (0.028 g, 1.02 x $10^{-4}$ mol), Vazo® 52 (15 mg, 6.04 x $10^{-5}$ mol).

Film D: methyl acrylate (3.20 g, 0.0368 mol), lithium N-(trifluoromethanesulfonyl)-2-(4-ethenylphenoxy) tetrafluoroethanesulfonimide (1.76 g, .0041 mol), diallyldiglycol carbonate (0.014 g, 5.11 x $10^{-5}$ mol), Vazo® 52 (15 mg, 6.04 x $10^{-5}$ mol).

The films were dried under vacuum at 85°C. TGA ($N_2$, 10°C/min): onset of decomposition at 140°C for all films. DSC ($N_2$, -100 to 180°C, 10°C/min): no $T_m$ detected. Anal. Found: Film A: % C 33.88, 33.80; % H 3.31, 3.24; % N 5.35, 5.36; % F 22.50, 22.36; % S 11.20, 10.89; % Li 1.09, 1.10; Film B: % C 34.12, 34.31; % H 3.43, 3.31; % N 5.67, 5.78; % F 22.04, 22.33; % S 11.03, 10.97; % Li 1.13, 1.15; Film C: % C 33.21, 33.28; % H 3.20, 3.18; % N 5.75, 5.78; % F 23.44, 23.10; % S 10.97, 10.99; % Li 1.14, 1.18; Film D: % C 33.72, 33.79; % H 3.22, 3.25; % N 5.56, 5.55; % F 23.36, 23.23; % S 12.76, 12.52; % Li 1.18, 1.14.

EXAMPLE 42

Copolymerization and Crosslinking of Lithium N-(Trifluoromethane-sulfonyl)-2-(4-ethenylphenoxy) tetrafluoroethanesulfonimide, Methvl Acrylate, and Diallyldiglycol Carbonate

Films were prepared in the following manner. The methyl acrylate, lithium N-(trifluoromethanesulfonyl)-2-(4-ethenylphenoxy)tetrafluoroethanesulfonimide, and carbonate monomers were placed in a vial with 1 mL of DMF and a stir bar. The initiator was added and stirred to dissolve. The solution was pipetted into a Teflon® petri dish and into squares on a piece of Teflon®-coated glass. The films were heated on a slide warmer at 40°C for 6 hours, left at room temperature overnight, and then warmed at 45°C for 8 hrs. The amounts of the monomers and initiator are given below.

Film A: methyl acrylate (3.13 g, .036 mol), lithium N-(trifluoromethanesulfonyl)-2-(4-ethenylphenoxy) tetrafluoroethanesulfonimide (1.76 g, .00403 mol), diallydiglycol carbonate (0.111 g, 4.05 x $10^{-4}$ mol), Vazo® 52 (15 mg, 6.04 x $10^{-5}$ mol).

Film B: methyl acrylate (3.17 g, .036 mol), lithium N-(trifluoromethanesulfonyl)-2-(4-ethenylphenoxy) tetrafluoroethanesulfonimide (1.78 g, .00407 mol), diallydiglycol carbonate (0.0557 g, 2.03 x $10^{-4}$ mol), Vazo® 52 (15 mg, 6.04 x $10^{-5}$).

Film C: methyl acrylate (3.18 g, 0.0365 mol), lithium N-(trifluoromethanesulfonyl)-2-(4-ethenylphenoxy) tetrafluoroethanesulfonimide (1.78 g, .00407 mol), diallydiglycol carbonate (0.028 g, 1.02 x $10^{-4}$ mol), Vazo® 52 (15 mg, 6.04 x $10^{-5}$).

Film D: methyl acrylate (3.20 g, 0.0368 mol), lithium N-(trifluoromethanesulfonyl)-2-(4-ethenylphenoxy) tetrafluoroethanesulfonimide (1.76 g, .0041 mol), diallydiglycol carbonate (0.014 g, $5.11 \times 10^{-5}$ mol), Vazo® 52 15 mg ($6.04 \times 10^{-5}$).

Films were dried under vacuum. TGA ($N_2$, 10°C/min): onset of decomposition at 150°C for all films. DSC ($N_2$, -100 to 100°C, 10 °C/min): no $T_m$ detected.

EXAMPLE 43

Copolymerization of Lithium 2-(4-ethenylphenoxy)tetrafluoroethanesulfonate, Lithium N-(Trifluoromethanesulfonyl)-2-(4-ethenylphenoxy)-tetrafluoroethanesulfonimide, and Butyl Acrylate

In a glovebox, 1.0 g (.00327 mol) of lithium 2-(4-ethenylphenoxy)tetrafluoroethanesulfonate, 1.43 g (.0033 mol) of lithium N-(trifluoromethanesulfonyl)-2-(4-ethenylphenoxy)tetrafluoroethanesulfonimide, and 7.56 g (0.059 mol) of butyl acrylate were combined in a Schlenk tube with a stir bar. followed by the addition of 45 mg ($1.81 \times 10^{-4}$ mol) of Vazo® 52 and stirred to dissolve. The flask was placed under argon and heated at 40°C for 24 hours, followed by 45°C for 24 hours. The resultant polymer was dissolved in acetone/THF, precipitated into water (2x). The polymer was then dissolved into acetone/THF (3:1) and precipitated into hexane (2x). The polymer was dried under vacuum at 60°C to obtain 3.3 g (33%) yield. Films could be obtained by dissolving 0.5 g of polymer in 20 mL 3:1 acetone/THF and casting the solution into a 5.5 cm diameter Teflon® petri dish. TGA ($N_2$, 10°C/min): onset of decomposition at 300°C. DSC ($N_2$, -100 to 250°C, 10°C/min): no $T_m$ detected. $^{19}F$ NMR (d-THF) δ: -80.2 (3F), -81.2 (2F), -82.6 (2F), -117.1 (2F), -118.4 (2F) ppm. $^{19}F$ NMR integration indicated equal amounts of **1** and **2**. $^{13}C$ NMR (d-THF) δ: 175.0, 64.9, 42.5, 36.2 (b), 31.9, 20.2, 14.3 ppm. $^{1}H$ NMR (d-THF) δ: 7.1, 4.0, 2.9, 2.3, 1.8, 1.7-1.2 (b), 0.9 ppm. $^{1}H$ NMR integration indicated 7 mol % ionomeric content based on integration of aromatic signal to methyl signal of butyl acrylate. Anal. Found: % C 8.24, 58.52; % H 8.32, 8.12; % N 0.48, 0.41; % F 5.69, 5.89; % Li 0.33, 0.35; % S 3.70, 3.47.

EXAMPLE 44

Synthesis of (ar-Vinylbenzyl)trimethylammonium 2-(4-ethenylphenoxy)tetrafluoroethanesulfonate

Lithium 2-(4-ethenylphenoxy)tetrafluoroethanesulfonate (3.06 g, 0.01 mol) was dissolved in 10 mL deionized water. To this was added a solution of 2.12 g (0.01 mol) (ar-vinylbenzyl)trimethylammonium chloride (Aldrich, mixture of meta and para isomers) in 10 mL deionized water. An oily gum precipitated instantly which turned into a crystalline solid on further stirring. The solid was collected, washed with 20 mL of water and dried at 40°C under vacuum to give 4.52 g (95%) of the title salt which was soluble in THF, DMF and methanol. $^{1}H$ NMR (δ, $CD_3OD$) 3.08 (s, 9H), 4.50 (s, 2H), 5.25 (d, 1H), 5.35 (d, 1H), 5.5 (d, 1H), 5.88 (d, 1H), 6.60 - 6.80 (2dd, 2H), 7.23 (d, 2H), 7.55 (m, 6H); $^{19}F$ NMR (δ, $CD_3OD$) -81.07 (2F), -116.66 (2F). Anal. Calcd. for $C_{22}H_{25}F_4NO_4S$: C, 55.57; H, 5.30; N, 2.95; S, 6.74; F. 15.98. Found: C, 54.27; H, 5.09; N, 2.83; S, 7.19; F, 15.79.

EXAMPLE 45

Synthesis of Synthesis of (ar-Vinylbenzyl)trimethylammonium N-(trifluoromethanesulfonyl)-2-(4-ethenylphenoxy) tetrafluoroethanesulfonimide

Lithium N-(trifluoromethane-sulfonyl)-2-(4-ethenylphenoxy)tetrafluoroethanesulfonimide (8.75 g, 0.02 mol) was dissolved in 20 mL of deionized water and cooled in an ice water bath. To this was added a solution of 4.24 g (0.02 mol) (ar-vinylbenzyl)trimethylammonium chloride (Aldrich, mixture of meta and para isomers) in 20 mL deionized water. A clear gum was immediately deposited. The aqueous solution was decanted and the gum was washed with water. Drying under vacuum at 30°C occured with severe foaming. The product was dissolved in 50 mL of methanol, filtered into a 300 mL round bottom flask and concentrated on a rotary evaporator to clear syrup which was dried for 16 hr at 30°C and 0.05 mm. The product weighed 11.5 g (95%). $^{1}H$ NMR (δ, $CD_3OD$) 3.1 (s, 9H), 4.47 (s, 2H), 5.25 (d, 1H), 5.35 (d, 1H), 5.76 (d, 1H), 5.87 (d, 1H), 6.68-6.81 (2dd, 2H), 7.20 and 7.50 (AB quartet, 4H), 7.42 - 7.50 (m, 4H); $^{19}F$ NMR (δ, $CD_3OD$) -78.91(3F), -79.77 (2F), -115.27 (2F).

EXAMPLE 46

Copolymerization of Lithium 2-(4-ethenylphenoxy)tetrafluoroethanesulfonate, (ar-Vinylbenzyl)trimethylammonium 2-(4-ethenylphenoxy)-tetrafluoroethanesulfonate and Methyl Methacrylate

A polymer tube was charged with 3.06 g (0.01 mol) of lithium 2-(4-ethenylphenoxy)tetrafluoroethanesulfonate, 2.38 g (0.005 mol) of the product from Example 44 and 20 mL of DMF. This solution was placed under pump vacuum until about 1 mL of solvent had evaporated to remove traces of methanol from the salts. Methyl methacrylate (8.5 g, 0.085 mol) and 0.05 g Vazo® 52 were added and the solution was subjected to three freeze, evacuate and thaw cycles. It was then heated for 20 hr in an oil bath at 50°C. The solution was cooled, diluted with 80 mL DMF and poured into water. This gave a gel which could not be filtered. The solvents were removed at 49°C and full pump vacuum to give 16.5 g of residue. This material was washed with 2 X 100 mL water, agitating for several hours with each portion. The water insoluble material was dried under high vacuum at 48°C giving 9.92 g of polymer. This material was soluble in methanol, acetone, DMF and DMSO and a clear brittle film could be cast from DMF. From the $^{13}C$ NMR spectrum of the polymer in DMF, it was calculated that it contained 81 mole % MMA units, 14 mole % $OCF_2CF_2SO_3^-$ units and 5 mole % $CH_2NMe_3$ units from integration of peaks at 17 ppm (MMA methyl), 118.4 + 122.3 ($CF_2$) and 68.8 ($CH_2N$). Anal. Found: C. 52.98; H, 6.26; F, 7.56; Li, 0.45; S, 3.66.

EXAMPLE 47

Copolymerization of Lithium N-(trifluoromethanesulfonyl)-2-(4-ethenylphenoxy)tetrafluoroethanesulfonimide, (ar-Vinylbenzyl)trimethylammonium N-(trifluoromethanesulfonyl)-2-(4-ethenylphenoxy)tetrafluoroethanesulfonimide and Methyl Methacrylate

A 50 mL polymer tube was cooled in ice water and charged with 2.19 g (0.005 mole) of lithium N-(trifluoromethanesulfonyl)-2-(4-ethenylphenoxy)-tetrafluoroethanesulfonimide and 10 mL of ice water. After the salt dissolved, a solution of 1.06 g (0.005 mole) of (ar-vinylbenzyl)trimethylammonium chloride in 10 mL of ice water was added resulting in immediate precipitation of a sticky gum. The water was decanted and the gum was stirred with 10 mL of ice water with a spatula. The gum was dried briefly under pump vacuum, then dissolved in 20 mL DMF. Lthium N-(trifluoromethanesulfonyl)-2-(4-ethenylphenoxy)-tetrafluoroethanesulfonimide (4.37 g, 0.01 mole) was added, followed by 8.5 g of methyl methacrylate and 0.05 g Vazo® 52. The solution was deoxygenated by 4 freeze/pump/thaw cycles, then heated in an oil bath for 22 hr at 50°C. An additional 20 mL DMF were added and the mixture was warmed to give a clear solution. Polymer was precipitated by adding the DMF solution to excess ether. The precipitate was washed in a blender with ether, then dried at 100°C and 0.1 mm to give 14.86 g of white solid. NMR showed that the solid still contained DMF so it was reprecipitated from acetone into ether and dried giving 12.45 g (78%) of white solid. $^1H$ NMR (δ, acetone-d6) 0.6 - 3.8 (multiplets), 4.60 (bs), 7.2-7.5 (b). By integration of the aliphatic versus benzyl versus aromatic peaks, calculate the polymer contains lithium N-(trifluoromethanesulfonyl)-2-(4-ethenylphenoxy)tetrafluoroethanesulfonimide (9.8 mole %), (ar-vinylbenzyl)-trimethylammonium N-(trifluoromethanesulfonyl)-2-(4-ethenylphenoxy)-tetrafluoroethanesulfonimide (4.6 mole %) and MMA (85.5 mole %). $^{19}F$ NMR (δ, acetone-d6) -78.69(3F), -79.47 (2F), -115.30 (2F). Anal. Found: C, 48.79; H, 5.52; N, 2.06, F, 12.22, Li, 0.40; S, 6.30. A film cast from acetone was clear and stiff.

EXAMPLE 48

Co-curing of Lithium 2-(4-ethenylphenoxy)tetrafluoroethanesulfonate with Hydrin®T Elastomer

A mixture of 2.5 g lithium 2-(4-ethenylphenoxy)tetrafluoroethanesulfonate, 5.0 g Hydrin®T and 50 mL of DMF was mixed in a glass jar on rollers until the solution was homogeneous. Benzoyl peroxide (0.3 g) was added and mixing was continued for 2 hr. A portion (15 mL) of this solution was poured into each of 3 casting molds (Teflon®, 2 7/16 inches square). The molds were place in a vacuum oven under nitrogen purge at room temperature overnight, then heated to 70°C for 48 hr under vacuum with a slight nitrogen purge. The resulting films were readily peeled from the mold and were slightly yellow, highly flexible, tough and without visible phase separation. One film was weighed and immersed in THF for 24 hr at room temperature. This film was observed to swell to several times its size but retained its shape. It was removed from the THF, patted dry with paper towels and weighed. The original weight (1.95 g) had increased to 7.77 g (% swelling = 298). After drying the film returned to its original size. The THF solution was concentrated on a rotary evaporator giving 0.17 g (8.7 %) extractables. Anal. Found: C, 43.46; H, 5.64; F, 7.05; S, 2.69; Li, 0.62. From the % S analysis, equivalent weight was calculated to be 1190.

EXAMPLE 49

Co-curing of Lithium N-(trifluoromethanesulfonyl)-2-(4-ethenylphenoxy)tetrafluoroethanesulfonimide with Hydrin®T Elastomer

The procedure of Example 48 was followed using 2.5 g of the sulfonimide salt in place of the lithium sulfonate. One film weighed 1.80 g. After soaking in THF for 24 hr, the film weighed 12.01 g (567% swelling). Concentration of the THF solution gave 0.57 g (29% extractables). Anal. Found: C, 40.40; H, 5.66: F, 5.77; Li, 0.27; S, 3.24. From the % S analysis, equivalent weight was calculated to be 988.

EXAMPLE 50

Polymerization of 3,5-di($CO_2CH_3$)-Ph-$OCF_2CF_2SO_3Li$ and Bis(hydroxyethyl)terephthalate

A 500 mL round bottom flask with mechanical stirrer and distillation head was charged with 28.5 g (0.0712 mol) of 3,5-di($CO_2CH_3$)-Ph-$OCF_2CF_2SO_3Li$ and 20 g of ethylene glycol. The flask was immersed in a tin/bismoth alloy bath, preheated to 200°C, and the contents were stirred until a homogenous solution was formed. The stirring was halted and 54.8 g (0.216 mol) of bis(hydroxyethyl)-terephthalate and 0.02 g of titanium (IV) isopropoxide were added. The system was flushed with nitrogen by alternatively evacuating and filling to one atmosphere several times. The stirrer speed was set to 50 rpm and the bath temperature was increased to 240°C over 1 hr resulting in a slow distillation. The bath temperature was gradually increased to 250°C and the system pressure decreased to 160 millitorr over 3.75 hr with continuing collection of distillate. The stirring torque increased from 24 to 180 during this period. The mixture was allowed to cool to room temperature and 82.1 g of solid and 19.2 g of distillate were isolated. The solid was dissolved in γ-butyrolactone and the solution was filtered and added to excess ether to precipitate a gum. The gum was dried at 135°C and 0.05 mm pressure. It was dissolved in about I-L of acetone and the solution was concentrated to 500 mL and added in small portions to excess ether. The precipitated polymer was dried at room temperature and 0.05 mm giving 63.8 g of product. Intrinsic viscosity (1:1 $CH_2Cl_2$:$CF_3COOH$) = 0.334; Tg (DSC) = 84°C. Proton NMR (acetone-d6) shows peaks at δ 8.5 and 8.0 assigned to one proton from the sulfonate-substituted aromatic groups and two protons from the sulfonate-substituted groups plus four protons from the terephthalate units. By integration, the ratio of these groups is calculated to be 1:3 in excellent agreement with the starting material ratio. Fluorine (acetone-d6) shows peaks of equal area at δ -81.2 and δ -116.7.

EXAMPLE 51

Polymerization of Bis(hydroxyethyl)terephthalate and

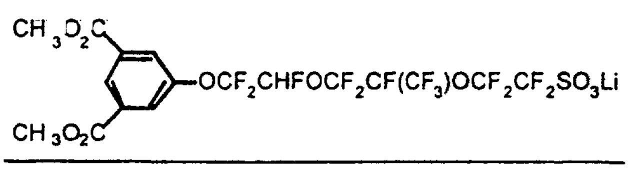

The procedure of example 50 was followed using 33.0 g (0.05 mol) of the lithium sulfonate monomer from example 9, 20.3 g (0.08 mol) of bis(hydroxyethyl)terephthalate, 30.0 g of ethylene glycol and 0.02 g of titanium (IV) isopropoxide. Final torque on the stirrer motor was 150. Isolated was 51.5 g of polymer and 27.5 g of distillate. The polymer was dissolved in acetone. This solution was filtered and the filtrate added slowly to excess ether. The resulting stringy polymer was collected and dried giving 31.8 g of off-white foam. Proton NMR was similar to that described in example 50 with integration of appropriate peaks indicating a ratio of 1.7:1 for the terephthalate to sulfonate-substituted units. F NMR (δ, acetone-d6) -78.5 to - 79.7 (m, 5 F, CF3 + CF2), - 81.7 to - 86.7 (m, 4F, $2CF_2$), -117.2 (s 2F, $1CF_2$), -144.1 to - 145.1 (m, 2F, CF + CFH).

EXAMPLE 52

The solution cast ionomer film of Example 14 was dried in a recirculating nitrogen oven (Electric Hotpack Company, Inc., Model 633, Philadelphia, PA) at 110°C for 48 hours.

The dried film was transferred to a sealed container while still warm and conveyed to a glove box having a positive pressure of dry nitrogen applied thereto, wherein the membrane was removed from the sealed container and

allowed to come to room temperature. The membrane was then cut into several sections 1.0 cm x 1.5 cm in size.

A 1:1 by volume mixture of ethylene carbonate (EC, 98%, Aldrich Chemical Co., Inc., Milwaukee, WI) and-dimethyl carbonate (DMC, 99%, Alfa Aesar, Ward Hill, MA) was deposited by pipette on to the top surface of a cooled 1.0 cm x 1.5 cm film specimen in an amount equal to 133% of the weight of the film sample. After waiting thirty minutes, the ionic conductivity was measured.

The solvent-treated film was blotted dry and positioned into the conductivity cell. Cell impedance was determined over the range of 10 Hz to 100,000 Hz, and the value with zero phase angle in the higher frequency range (usually 500-5000 Hz) was ascribed to the bulk sample resistance in Ohms. The raw resistance value was then converted to conductivity, in S/cm, using the cell constant and liquid-swollen film thickness. The ionic conductivity was found to be 1.03 x $10^{-4}$ S/cm at 23°C.

EXAMPLE 53

Several grams of the dry lithium salt of Example 3 herein were placed into a glove box having a positive pressure of dry nitrogen applied thereto. The salt was combined with the EC/DMC mixture of Example 52 to form a 0.5 M solution. The liquid conductivity of this solution was measured to be 1.72 x $10^{-3}$ S/cm at 23°C.

EXAMPLE 54

Several grams of the dry lithium salt of Example 7 herein were placed into a glove box having a positive pressure of dry nitrogen applied thereto. The salt was combined with the EC/DMC mixture of Example 52 to form a 1.0 M solution. This solution was diluted by 50% to create a 0.5 M solution, the liquid conductivity of which was measured to be 4.30 x $10^{-3}$ S/cm at 23°C.

EXAMPLE 55

The ionomer film of Example 16 was dried in a recirculating nitrogen oven (Electric Hotpack Company, Inc., Model 633, Philadelphia, PA) at 100°C for 48 hours.

A cooled 1.0 cm by 1.5 cm membrane sample was immersed into deionized water for a period of several hours. The ionic conductivity was determined as in Example 52, except that the specimen was tested outside the glove box, and found to be 1.588 x $10^{-3}$ S/cm at 23°C.

EXAMPLE 56

The ionomer film of Example 49 (E93722-105-5A) was dried in a recirculating nitrogen oven (Electric Hotpack Company, Inc., Model 633, Philadelphia, PA) at 75°C under vacuum for 48 hours then transferred to a nitrogen-purged Vacuum Atmospheres dry box.

A cooled 1.0 cm by 1.5 cm membrane sample was immersed into an excess of propylene carbonate (Selectipur, EM Industries) for a period of five minutes. After this period, the membrane sample was blotted dry on its surface with a paper towel and its weight uptake and ionic conductivity were measured. The weight uptake of propylene carbonate was equal to 554% by weight of the dry sample. The solvent swollen film was free standing and easy to handle. The ionic conductivity was determined as in Example 52 and found to be 2.59 x $10^{-4}$ S/cm at 23°C.

EXAMPLE 57

The ionomer film of Example 30 (E92207-78) was obtained as described there and transferred directly to a nitrogen-purged Vacuum Atmospheres dry box.

A cooled 1.0 cm by 1.5 cm membrane sample was immersed into an excess of propylene carbonate (Selectipur, EM Industries) for a period of ten minutes. After this period, the membrane sample was blotted dry on its surface with a paper towel and its weight uptake and ionic conductivity were measured. The weight uptake of propylene carbonate was equal to 366% by weight of the dry sample. The solvent swollen film was free standing and easy to handle. The ionic conductivity was determined as in Example 52 and found to be 4.12 x $10^{-4}$ S/cm at 23°C.

EXAMPLES 58 AND 59

A slurry was prepared by mixing 65 g of MCMB 25-28 graphite (Osaka Gas Co., Ltd, Osaka, Japan), 10 g Kynar Flex® 2801 polyvinylidene fluoride-hexafluoropropylene copolymer (Elf Atochem, Philadelphia, PA), 3.5 g Super P carbon black (MMM S.A. Carbon, Brussels, Belgium), 21.5 g dibutyl phthalate plasticizer, and 150 g of acetone as

casting solvent. The slurry was then cast into a graphite electrode film 0.111-0.126 mm in thickness, using a doctor blade with a ca. 0.500 mm gate height.

A layered structure was formed by placing a piece of the electrode film between brass foils of about 0.127 mm thickness, employing 0.095 mm thick shims between the foils to prevent excessive pressure on the electrode. In order to better consolidate the electrode film, the layered structure was passed through a Western Magnum XRL 120 Laminator set to a temperature of 115°C, and a pressure of 20 psig. After consolidation, the electrode was removed from between the brass foils and the thickness determined to be 0.108 mm.

The dibutyl phthalate was then extracted from the consolidated electrode film using diethylether after which two circular pieces of about 15.9 mm diameter were cut from the extracted electrode film, the circular pieces being dried in an antechamber and introduced into a glove-box under Argon atmosphere.

In a glove box under an Argon atmosphere, 0.725 g of $C_2H_3C_6H_4OCF_2CF_2SO_3Li$ prepared in the manner of Example 3 was placed in a 10 ml volumetric flask to which was added a 1:1 by weight mixture of ethylene carbonate and dimethyl carbonate adding EC/DMC 1:1 (by weight) and the mixture so formed stirred at room temperature for ca. 20 minutes to form an electrolyte solution, and the stirring time was 10 minutes. The conductivity of the electrolyte solution was determined to be 1.131 mS/cm at room temperature, employing the method hereinabove described.

The two 15.9 mm diameter specimen of the dried graphite electrode film and two 15.9 mm diameter piece of Celgard® 3501 microporous polypropylene 24 micrometers in thickness were soaked in the electrolyte solution in separate closed vials in the glove-box for twenty minutes each.

Two size 2325 electrochemical coin cells were formed by employing in each the soaked graphite electrode as the positive electrode, the soaked Celgard® film as the separator, and a 15.9 mm diameter circle of Li metal foil 0.333 mm in thickness as the negative electrode.

Each coin cell was sealed and discharged with constant current of 0.5 mA to a voltage of 0.01 V, at which point the voltage was held constant until the current dropped below 0.05 mA. Each cell was then charged at a constant current of 0.5 mA to 1.1 V, and then the voltage was held constant at 1.1 V until the charging current dropped below 0.05 mA.

The positive electrode capacities on charge and discharge for the two coin cells are shown in Table 1.

Table 1:

| Positive Electrode Capacities | | |
|---|---|---|
| | Example 58 | Example 59 |
| Wt. of positive electrode,g | 0.0290 | 0.0289 |
| Wt. Of electrolyte,g | 0.0406 | 0.0582 |
| Discharge (mAh/g of graphite) | 306 | 238 |
| Charge (mAh/g of graphite) | 272 | 204 |

EXAMPLES 60 AND 61

The procedures of Examples 58 and 59 were repeated with the exceptions here noted. The electrode film specimens and separator were 12.7 mm in diameter, the positive electrode films were 0.095 mm in thickness. The electrolyte solution was formed as in Examples 58 and 59 except that 0.660 g of $C_2H_3C_6H_4OCF_2CF_2SO_3Li$, was employed. The conductivity of the electrolyte solution was determined to be 1.249 mS/cm at room temperature under Argon, employing the method hereinabove described.

The positive electrode capacities on charge and discharge for the two coin cells are shown in Table 2.

Table 2:

| Positive Electrode Capacities | | |
|---|---|---|
| | Example 60 | Example 61 |
| Wt. of positive electrode,g | 0.0167 | 0.0166 |
| Wt. Of electrolyte,g | 0.0341 | 0.0331 |
| Discharge (mAh/g of graphite) | 306 | 331 |
| Charge (mAh/g of graphite) | 273 | 294 |

## Claims

1. A polymer comprising pendant groups comprising the radical described by the formula I(a) or I(b)

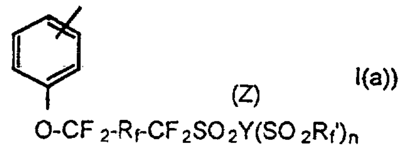

I(a))

or

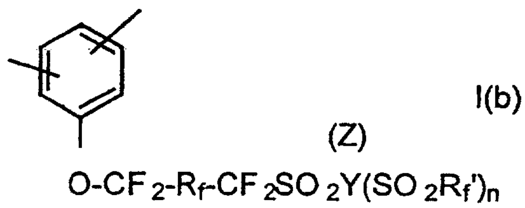

I(b)

wherein $R_f$ is a bond or is a fluoroalkylene group of from 1 to about 10 carbon atoms, optionally substituted by one or more ether oxygens and one or more hydrogen atoms, Y is N, O, C, $R_f'$ is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

2. A compound described by the formula (II)

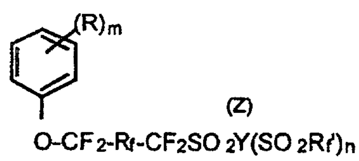

(II)

wherein m = 0, 1 or 2 and when m = 1 R is a polymerizable group or bromo or iodo, and when m = 2, R represents polymerizable groups, or bromo groups, or iodo groups, which are optionally the same, $R_f$ is a bond or is a fluoroalkylene group of from I to about 10 carbon atoms, optionally substituted by one or more ether oxygens and one or more hydrogen atoms, Y is N, O, C, $R_f'$ is a fluoroalkyl group of from 1 to about 10 carbon atoms, optionally substituted by one or more ether oxygens and one or more hydrogen atoms, n = 0, 1 or 2, n = 0, 1 or 2, with the proviso that n = 0 when Y = O, n = 1 when Y = N and n = 2 when Y = C, and Z is hydrogen or a univalent metal.

3. A process comprising:

reacting alkali metal salts of substituted phenols described by the formula (III) with 1,2-dibromotetrafluoroethane to make compounds described by the formula (IV)

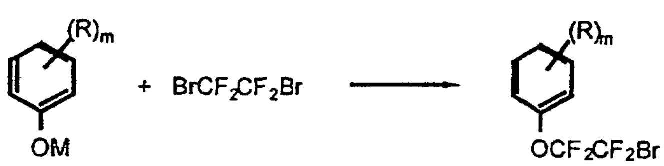

(III) (IV)

wherein in is 0, 1 or 2 and R is bromo, iodo, $CO_2R'$ or $NO_2$, R' is an alkyl group of 1 to 10 carbon atoms and M is an alkali metal. reacting the compound described by formula (IV) with a sulfinating reagent to form an alkali metal sulfinate described by structure (V)

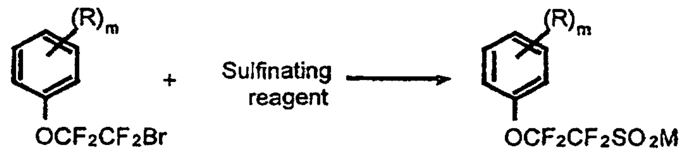


(IV) (V)

Reacting the alkali metal sulfmate of structure (V) with elemental chlorine or bromine to give the corresponding sulfonyl chloride or bromide described by structure (VI) wherein X = Cl or Br;

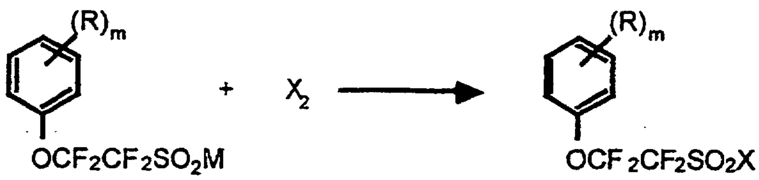

(V) (VI)

4. An ionically conductive composition comprising the ionomer of Claim 1 and a liquid imbibed therewithin or an ionically conductive composition comprising the compound described by the formula (II) and a liquid imbibed therewithin.

5. An ion exchange membrane comprising an ionomer comprising pendant groups comprising the radical described by the formula

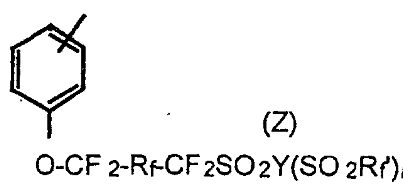

I(a))

or

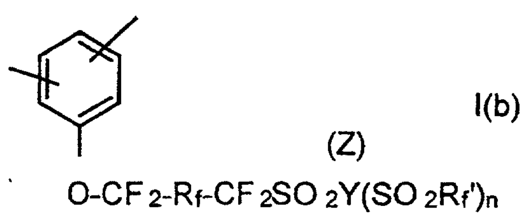

I(b)

wherein $R_f$ is a borid or is a uoro ene group of from 1 to about 10 carbon atoms, optionally substituted by one or more ether oxygens and one or more hydrogen atoms, Y is N, O, or C, $R_f'$ is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

6. An electrochemical cell comprising a cathode, an anode and a separator, at least one of which comprises an

ionomer comprising pendant groups comprising the radical described by the formula

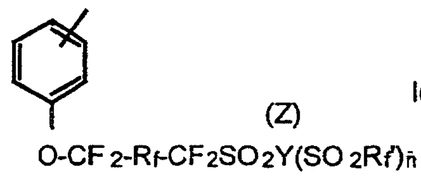

I(a))

or

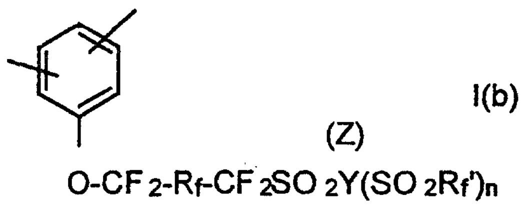

I(b)

wherein $R_f$ is a bond or is a fluoroalkylene group of from 1 to about 10 carbon atoms, optionally substituted by one or more ether oxygens and one or more hydrogen atoms, Y is N, O, or C, $R_f'$ is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

**7.** An electrochemical cell comprising an anode, a cathode, a separator, and a conductive composition comprising the compound described by the formula (II) and a liquid.

**8.** An electrode comprising an electroactive material and an ionomer comprising pendant groups comprising the radical described by the formula

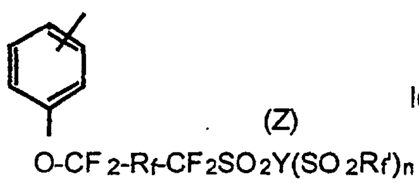

I(a))

or

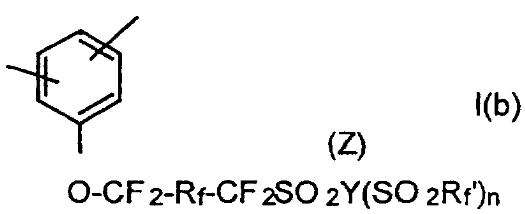

I(b)

wherein $R_f$ is a bond or is a fluoroalkylene group of from 1 to about 10 carbon atoms, optionally substituted by one or more ether oxygens and one or more hydrogen atoms, Y is N, O, or C, $R_f'$ is a fluoroalkyl group of from 1 to about 10 carbon atoms, optionally substituted by one or more ether oxygens and one or more hydrogen atoms, n = 0, 1 or 2 with the proviso that n = 0 when Y = O, n = 1 when Y = N and n = 2 when Y = C, and Z is hydrogen or a univalent metal.

9. The ionically conductive composition of Claims 4 in a form selected from the group consisting of a film, sheet and gel.

10. The ionically conductive composition of Claim 9 further comprising a microporous electrically insulating polymer film or sheet within the micropores of which the gel is imbibed.

11. The compound of Claim 2 wherein R is in the para position.

12. The polymer of Claim 1 or compound of Claim 2 wherein $R_f$ is $CF_2$ and $R_f'$ is $CF_3$ or $C_2F_5$.

13. The polymer of Claim 1 wherein the backbone is cross-linked.

14. The polymer of Claim 1 or compound of Claim 2 wherein Z is $Li^+$.

15. The polymer of Claim 1 or compound of Claim 2 wherein Y is N.

16. The polymer of Claim 1 or compound of Claim 2 wherein Y is O.

17. The compound of Claim 2 wherein R is $-CH=CH_2$ and m=1.

18. The compound of Claim 2 wherein R is $-CO_2R'$ and m=2, where R' is an alkyl radical having 1-10 carbons.

19. The ionically conductive composition of Claim 4 wherein the liquid is selected from the group comprising organic carbonates, diesters, and esters, and mixtures thereof.

20. The ionically conductive composition of Claim 19 wherein the liquid is a mixture of a cyclic organic carbonate and a diester or wherein the liquid is a mixture of ethylene carbonate and dimethyl succinate.

**Patentansprüche**

1. Polymer, umfassend Seitengruppen, umfassend das Radikal, beschrieben durch die Formel I(a) oder I(b)

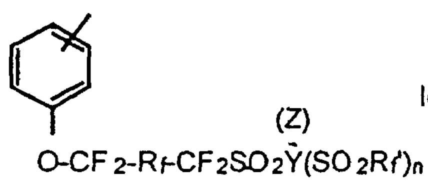

I(a))

oder

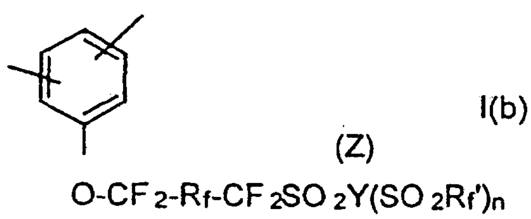

I(b)

worin $R_f$ eine Bindung darstellt oder eine Fluoralkylengruppe aus von 1 bis ca. 10 Kohlenstoffatom(en), optional substituiert durch einen oder mehrere Ether-Sauerstoff(e) und ein oder mehrere Wasserstoffatom(e) darstellt, Y für N, O, C steht, Rf' eine Fluoralkylgruppe aus von 1 bis ca. 10 Kohlenstoffatom(en), optional substituiert durch einen oder mehrere Ether-Sauerstoff(e) und ein oder mehrere Wasserstoffatom(e) darstellt, n = 0, 1 oder 2 ist, unter der Voraussetzung, dass n = 0, wenn Y = O ist, n = 1, wenn Y = N ist und n = 2, wenn Y = C ist und Z für Wasserstoff oder ein einwertiges Metall steht.

**2.** Verbindung, beschrieben durch die Formel (II)

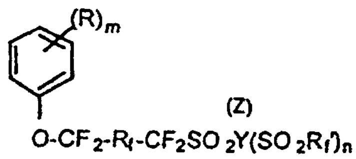

(II)

worin m = 0, 1 oder 2 ist und wenn m = 1 ist, R eine polymerisierbare Gruppe oder Bromo oder Iodo darstellt, und wenn m = 2 ist, R polymerisierbare Gruppen oder Bromo-Gruppen oder Iodo-Gruppen, die optional gleich sind, darstellt, $R_f$ eine Bindung darstellt oder eine Fluoralkylengruppe aus von 1 bis ca. 10 Kohlenstoffatom(en), optional substituiert durch einen oder mehrere Ether-Sauerstoff(e) und ein oder mehrere Wasserstoffatom(e) darstellt, Y für N, O, C steht, $R_f$' eine Fluoralkylgruppe aus von 1 bis ca. 10 Kohlenstoffatom(en), optional substituiert durch einen oder mehrere Ether-Sauerstoff(e) und ein oder mehrere Wasserstoffatom(e) darstellt, n = 0, 1 oder 2 ist, unter der Voraussetzung, dass n = 0, wenn Y = O ist, n = 1, wenn Y = N ist und n = 2, wenn Y = C ist und Z für Wasserstoff oder ein einwertiges Metall steht.

**3.** Verfahren umfassend:

zur Reaktion bringen von Alkalimetallsalzen von substituierten Phenolen, beschrieben durch die Formell (III) mit 1,2-Dibromtetrafluorethan zur Herstellung von Verbindungen, beschrieben durch die Formel (IV)

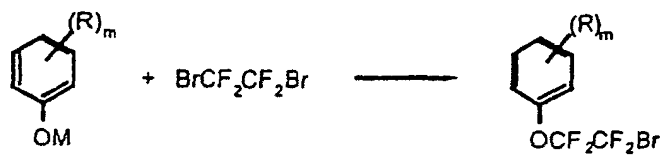

(III) (IV)

worin m für 0, 1 oder 2 steht und R Bromo, Iodo, $CO_2R'$ oder $NO_2$ darstellt, R' eine Alkylgruppe von 1 bis 10 Kohlenstoffatom(en) darstellt und M ein Alkalimetall darstellt,

zur Reaktion bringen der durch die Formel (IV) beschriebenen Verbindung mit einem Sulfinierungsreagenz zur Bildung eines Alkalimetallsulfinats, beschrieben durch die Struktur (V)

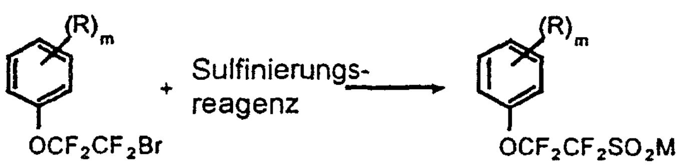


(IV) (V)

zur Reaktion bringen des Alkalimetallsulfinats der Struktur (V) mit elementarem Chlor oder Brom zum Erhalt des entsprechenden Sulfonylchlorids oder -bromids, beschrieben durch die Struktur (VI), worin X = Cl oder Br ist;

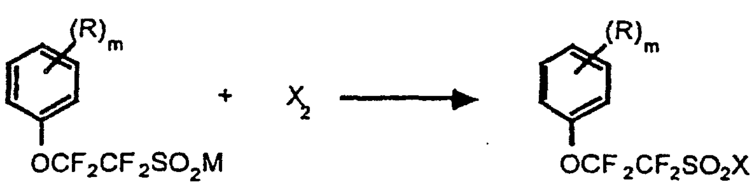

(V) (VI)

**4.** Ionisch leitfähige Zusammensetzung umfassend das Ionomer nach Anspruch 1 und eine darin imbibierte Flüssigkeit oder eine ionisch leitfähige Zusammensetzung, umfassend die Verbindung, beschrieben durch die Formel (II) und eine darin imbibierte Flüssigkeit.

**5.** Ionenaustauschmembran, umfassend ein Ionomer, umfassend Seitengruppen, umfassend das Radikal, beschrieben durch die Formel

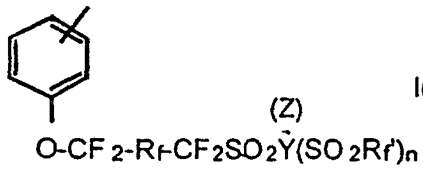

I(a))

oder

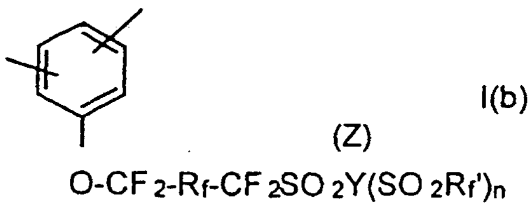

I(b)

worin $R_f$ eine Bindung darstellt oder eine Fluoralkylengruppe aus von 1 bis ca. 10 Kohlenstoffatom(en), optional substituiert durch einen oder mehrere Ether-Sauerstoff(e) und ein oder mehrere Wasserstoffatom(e) darstellt, Y für N, O oder C steht, $R_f$' eine Fluoralkylgruppe aus von 1 bis ca. 10 Kohlenstoffatom(en), optional substituiert durch einen oder mehrere Ether-Sauerstoff(e) und ein oder mehrere Wasserstoffatom(e) darstellt, n = 0, 1 oder 2 ist, unter der Voraussetzung, dass n = 0, wenn Y = O ist, n = 1, wenn Y = N ist und n = 2, wenn Y = C ist und Z für Wasserstoff oder ein einwertiges Metall steht.

**6.** Elektrochemische Zelle, umfassend eine Kathode, eine Anode und einen Separator, von denen mindestens eine (r) ein Ionomer umfasst, umfassend Seitengruppen, umfassend das Radikal, beschrieben durch die Formel

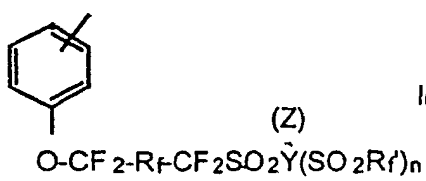

I(a))

oder

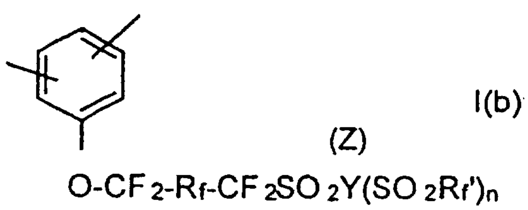

I(b)

worin $R_f$ eine Bindung darstellt oder eine Fluoralkylengruppe aus von 1 bis ca. 10 Kohlenstoffatom(en), optional substituiert durch einen oder mehrere Ether-Sauerstoff(e) und ein oder mehrere Wasserstoffatom(e) darstellt, Y für N, O oder C steht, $R_f$' eine Fluoralkylgruppe aus von 1 bis ca. 10 Kohlenstoffatom(en), optional substituiert durch einen oder mehrere Ether-Sauerstoff(e) und ein oder mehrere Wasserstoffatom(e) darstellt, n = 0, 1 oder 2 ist, unter der Voraussetzung, dass n = 0, wenn Y = O ist, n = 1, wenn Y = N ist und n = 2, wenn Y = C ist und Z

für Wasserstoff oder ein einwertiges Metall steht.

7. Elektrochemische Zelle, umfassend eine Anode, eine Kathode, einen Separator und eine leitfähige Zusammensetzung, umfassend die Verbindung, beschrieben durch die Formel (II) und eine Flüssigkeit.

8. Elektrode, umfassend ein elektroaktives Material und ein Ionomer, umfassend Seitengruppen, umfassend das Radikal, beschrieben durch die Formel

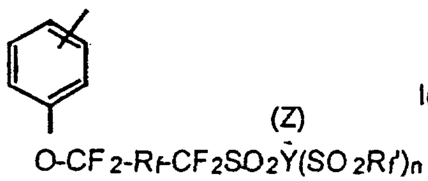

I(a))

oder

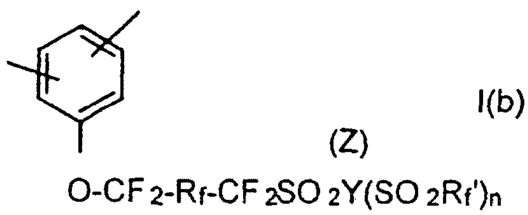

I(b)

worin $R_f$ eine Bindung darstellt oder eine Fluoralkylengruppe aus von 1 bis ca. 10 Kohlenstoffatom(en), optional substituiert durch einen oder mehrere Ether-Sauerstoff(e) und ein oder mehrere Wasserstoffatom(e) darstellt, Y für N, O oder C steht, $R_f'$ eine Fluoralkylgruppe aus von 1 bis ca. 10 Kohlenstoffatom(en), optional substituiert durch einen oder mehrere Ether-Sauerstoff(e) und ein oder mehrere Wasserstoffatom(e) darstellt, n = 0, 1 oder 2 ist, unter der Voraussetzung, dass n = 0, wenn Y = O ist, n = 1, wenn Y = N ist und n = 2, wenn Y = C ist und Z für Wasserstoff oder ein einwertiges Metall steht.

9. Ionisch leitfähige Zusammensetzung nach Anspruch 4 in einer Form, die aus der Gruppe ausgewählt ist, bestehend aus einem Film, einer Folie und einem Gel.

10. Ionisch leitfähige Zusammensetzung nach Anspruch 9, weiter umfassend eine(n) mikroporöse(n) elektrisch isolierende(n) Polymerfilm oder -folie, in dessen/deren Mikroporen das Gel imbibiert ist.

11. Verbindung nach Anspruch 2, worin sich R in der para-Stellung befindet.

12. Polymer nach Anspruch 1 oder Verbindung nach Anspruch 2, worin $R_f$ für $CF_2$ steht und $R_f'$ für $CF_3$ oder $C_2F_5$ steht.

13. Polymer nach Anspruch 1, worin die Hauptkette vemetzt ist.

14. Polymer nach Anspruch 1 oder Verbindung nach Anspruch 2, worin Z für $Li^+$ steht.

15. Polymer nach Anspruch 1 oder Verbindung nach Anspruch 2, worin Y für N steht.

16. Polymer nach Anspruch 1 oder Verbindung nach Anspruch 2, worin Y für O steht.

17. Verbindung nach Anspruch 2, worin R für $-CH=CH_2$ steht und m = 1 ist.

18. Verbindung nach Anspruch 2, worin R für $-CO_2R'$ steht und m = 2 ist, worin R' ein Alkylradikal mit 1 bis 10 Kohlenstoff (en) darstellt.

19. Ionisch leitfähige Zusammensetzung nach Anspruch 4, worin die Flüssigkeit aus der Gruppe ausgewählt ist, umfassend organische Carbonate, Diester und Ester und Gemische davon.

**20.** Ionisch leitfähige Zusammensetzung nach Anspruch 19, worin die Flüssigkeit ein Gemisch aus einem cyclischen organischen Carbonat und einem Diester darstellt oder worin die Flüssigkeit ein Gemisch aus Ethylencarbonat und Dimethylsuccinat darstellt.

### Revendications

**1.** Polymère comprenant des groupes pendants comprenant le radical décrit par la formule générale I(a) ou I(b):

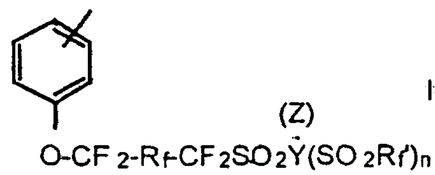

I(a))

ou

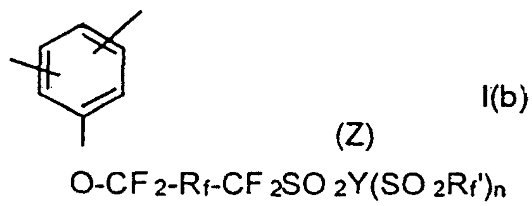

I(b)

dans laquelle $R_f$ est une liaison ou est un groupe fluoroalkylène de 1 à environ 10 atome(s) de carbone, éventuellement substitué par un ou plusieurs oxygène(s) d'éther et un ou plusieurs atome(s) d'hydrogène, Y est N, O, C, $R_f'$ est un groupe fluoroalkyle de 1 à environ 10 atome(s) de carbone, éventuellement substitué par un ou plusieurs oxygène(s) d'éther et un ou plusieurs atome(s) d'hydrogène, n = 0, 1 ou 2 sous réserve que n = 0 lorsque Y = O, n = 1 lorsque Y = N et n = 2 lorsque Y = C, et Z est l'hydrogène ou un métal monovalent.

**2.** Composé décrit par la formule générale (II)

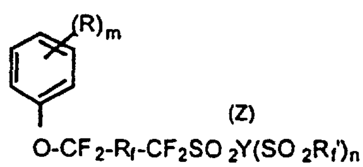

(II)

dans laquelle m = 0, 1 ou 2 et lorsque m = 1 R est un groupe polymérisable ou un groupe bromo ou iodo, et lorsque m = 2, R représente des groupes polymérisables, ou des groupes bromo ou des groupes iodo, qui sont éventuellement le même, $R_f$ est une liaison ou est un groupe fluoroalkylène de 1 à environ 10 atome(s) de carbone, éventuellement substitué par un ou plusieurs oxygène(s) d'éther et un ou plusieurs atome(s) d'hydrogène, Y est N, O, C, $R_f'$ est un groupe fluoroalkyle de 1 à environ 10 atome(s) de carbone, éventuellement substitué par un ou plusieurs oxygène(s) d'éther et un ou plusieurs atome(s) d'hydrogène, n = 0, 1 ou 2, sous réserve que n = 0 lorsque Y = O, n = 1 lorsque Y = N et n = 2 lorsque Y = C, et Z est l'hydrogène ou un métal monovalent.

**3.** Procédé comprenant les étapes consistant à:

faire réagir les sels de métal alcalin de phénols substitués décrits par la formule générale (III) avec du 1,2-dibromotétrafluoroéthane pour fabriquer des composés décrits par la formule générale (IV)

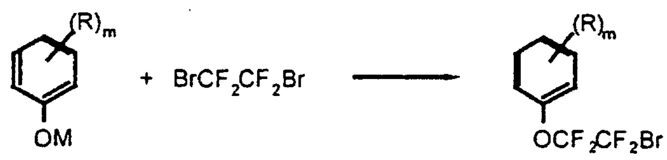


(III) (IV)

dans laquelle m a la valeur de 0, 1 ou 2 et R est un groupe bromo, ioda, un $CO_2R'$ ou $NO_2$, R' est un groupe alkyle de 1 à 10 atome(s) de carbone et M est un métal alcalin,
faire réagir le composé décrit par la formule générale (IV) avec un réactif de sulfinisation afin de former un sulfinate de métal alcalin décrit par la structure générale (V)

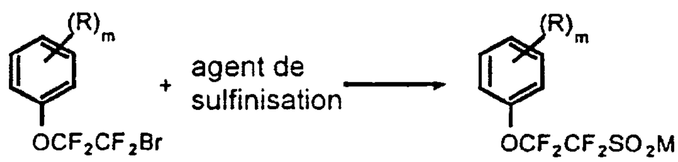


(IV) (V)

faire réagir le sulfinate de métal alcalin conforme à la structure générale (V) avec du chlore ou du brome élémentaire pour donner le chlorure ou le bromure de sulfonyle correspondant décrit par la structure générale (VI) dans laquelle X = Cl ou Br;

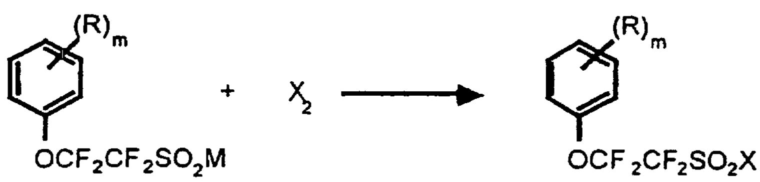


(V) (VI)

**4.** Composition ioniquement conductrice comprenant l'ionomère selon la revendication 1 et un liquide imbibé de celle-ci ou une composition ioniquement conductrice comprenant le composé décrit par la formule générale (II) et un liquide imbibé à l'intérieur de celle-ci.

**5.** Membrane échangeuse d'ions comprenant un ionomère comprenant des groupes pendants comprenant le radical décrit par la formule générale

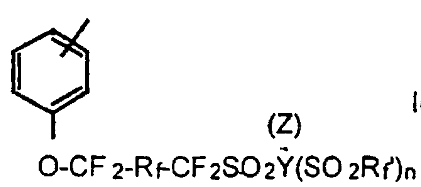

I(a))

ou

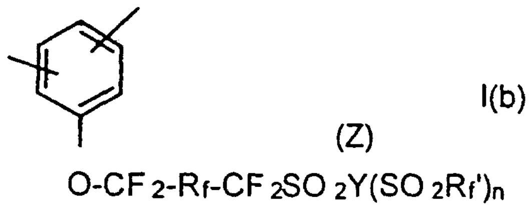

I(b)

dans laquelle $R_f$ est une liaison ou est un groupe fluoroalkylène de 1 à environ 10 atome(s) de carbone, éventuellement substitué par un ou plusieurs oxygène(s) d'éther et un ou plusieurs atome(s) d'hydrogène, Y est N, O, ou C, $R_f'$ est un groupe fluoroalkyle de 1 à environ 10 atome(s) de carbone, éventuellement substitué par un ou plusieurs oxygène(s) d'éther et un ou plusieurs atome(s) d'hydrogène, n = 0, 1 ou 2 sous réserve que n = 0 lorsque Y = O, n = 1 lorsque Y = N et n = 2 lorsque Y = C, et Z est l'hydrogène ou un métal monovalent.

6. Cellule électrochimique comprenant une cathode, une anode et un séparateur, au moins l'un de ceux-ci comprenant un ionomère comprenant des groupes pendants comprenant le radical décrit par la formule générale

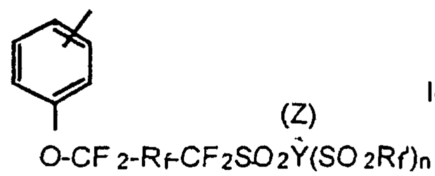

I(a))

ou

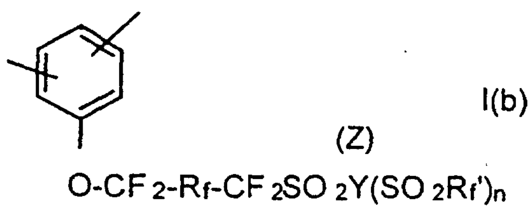

I(b)

dans laquelle $R_f$ est une liaison ou est un groupe fluoroalkylène de 1 à environ 10 atome(s) de carbone, éventuellement substitué par un ou plusieurs oxygène(s) d'éther et un ou plusieurs atome(s) d'hydrogène, Y est N, O, ou C, $R_f'$ est un groupe fluoroalkyle de 1 à environ 10 atome(s) de carbone, éventuellement substitué par un ou plusieurs oxygène(s) d'éther et un ou plusieurs atome(s) d'hydrogène, n = 0, 1 ou 2 sous réserve que n = 0 lorsque Y = O, n = 1 lorsque Y = N et n = 2 lorsque Y = C, et Z est l'hydrogène ou un métal monovalent.

7. Cellule électrochimique comprenant une anode, une cathode, un séparateur, et une composition conductrice comprenant le composé décrit par la formule générale (II) et un liquide.

8. Électrode comprenant un matériau électroactif et un ionomère comprenant des groupes pendants comprenant le radical décrit par la formule générale

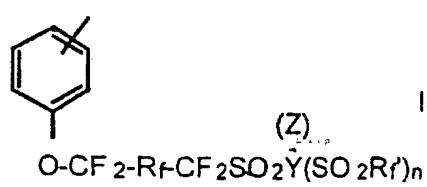

I(a))

ou

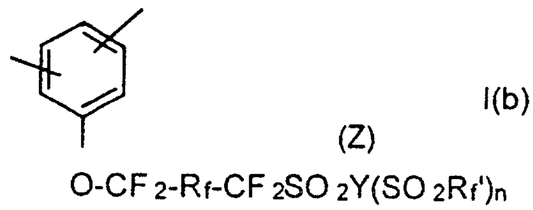

I(b)

dans laquelle $R_f$ est une liaison ou est un groupe fluoroalkylène de 1 à environ 10 atome(s) de carbone, éventuellement substitué par un ou plusieurs oxygène(s) d'éther et un ou plusieurs atome(s) d'hydrogène, Y est N, O ou C, $R_f'$ est un groupe fluoroalkyle de 1 à environ 10 atome(s) de carbone, éventuellement substitué par un ou plusieurs oxygène(s) d'éther et un ou plusieurs atome(s) d'hydrogène, n = 0, 1 ou 2 sous réserve que n = 0 lorsque Y = O, n = 1 lorsque Y = N et n = 2 lorsque Y = C, et Z est l'hydrogène ou un métal monovalent.

**9.** Composition ioniquement conductrice selon la revendication 4, sous une forme choisie au sein du groupe constitué d'un film, d'une feuille et d'un gel.

**10.** Composition ioniquement conductrice selon la revendication 9 comprenant en outre un film ou une feuille polymère microporeux(se) électriquement isolant(e) dans les micropores duquel (de laquelle) le gel est imbibé.

**11.** Composé selon la revendication 2, dans lequel R se situe en position para.

**12.** Polymère selon la revendication 1 ou composé selon la revendication 2, dans lequel $R_f$ est $CF_2$ et $R_f'$ est $CF_3$ ou $C_2F_5$.

**13.** Polymère selon la revendication 1, où le squelette est réticulé.

**14.** Polymère selon la revendication 1 ou composé selon la revendication 2, dans lequel Z est $Li^+$.

**15.** Polymère selon la revendication 1 ou composé selon la revendication 2, dans lequel Y est N.

**16.** Polymère selon la revendication 1 ou composé selon la revendication 2, dans lequel Y est O.

**17.** Composé selon la revendication 2, dans lequel R est $—CH{=}CH_2$ et m = 1.

**18.** Composé selon la revendication 2, dans lequel R est $—CO_2R'$ et m = 2, où R' est un radical alkyle ayant 1 à 10 atome(s) de carbone.

**19.** Composition ioniquement conductrice selon la revendication 4, dans laquelle le liquide est choisi au sein du groupe comprenant les carbonates organiques, les diesters et les esters, et les mélanges de ceux-ci.

**20.** Composition ioniquement conductrice selon la revendication 19, dans laquelle le liquide est un mélange de carbonate organique cyclique et d'un diester ou dans laquelle le liquide est un mélange de carbonate d'éthylène et de succinate diméthylique.